# EUROPEAN PATENT APPLICATION

(11) **EP 4 847 525 A1**
(43) Date of publication of application: **02.12.2026**
(21) Application number: 25779306.7
(22) Date of filing: 27.03.2025
(51) Int. Cl.: C07K 14/79, C07K 1/113, C07K 1/34, A61K 38/40, A61P 39/02, A61K 9/06, A61K 9/50, C08J 9/28, C08L 89/00, C08J 3/12

(54) **PREPARATION METHOD FOR AND USE OF LACTOFERRIN AND FORMULATION THEREOF**

(30) Priority: 28.03.2024 CN 202410362552; 28.03.2024 CN 202410362699; 28.03.2024 CN 202410362735
(71) Applicant: Myomed Technology (Shaoxing) Co., Ltd., Shaoxing, Zhejiang 312000 (CN)
(72) Inventor: ZHU, Yang, Hangzhou, Zhejiang 310012 (CN); ZHANG, Liwen, Hangzhou, Zhejiang 310012 (CN); REN, Tanchen, Hangzhou, Zhejiang 310012 (CN)
(74) Representative: Herzog IP Patentanwalts GmbH
(86) International application number: PCT/CN2025/085330
(87) International publication number: WO 2025/201452

(57) **Abstract**

A preparation method for and a use of a lactoferrin and a formulation thereof are provided. Iron saturation content of the lactoferrin is greater than 10 mg/g. The obtained lactoferrin can quickly reduce iron content in a microenvironment, thereby reducing iron to better treat iron overload diseases, which include but are not limited to myocardial ischemia-reperfusion injury, renal ischemia-reperfusion injury, cerebral infarction injury, and other diseases, and providing a stronger iron-deprivation antibacterial capability.

## Description

### Technical Field

This application belongs to the field of functional protein technologies, and relates to a preparation method for and a use of a lactoferrin and a formulation thereof.

### Background

Lactoferrin is a low-molecular-weight natural milk-derived glycoprotein with a relative molecular mass of approximately 80 kDa, and is widely present in milk, tears, and saliva. The lactoferrin has an iron-absorbing capability 300 times higher than that of transferrin, and may be used as a natural iron chelator. The sequence of the lactoferrin includes more than 700 amino acids. In terms of structure, the lactoferrin is composed of two globular lobes and the structure is stabilized by disulfide bonds. According to earlier theories, the center of each of the two globular lobes of the lactoferrin can chelate an iron ion, and the chelation site is stabilized with the help of externally provided carbonate (for example, there is a particular concentration of carbonate ion in body fluids), so that iron is not easily dissociated. In an environment with a pH lower than 4.0, the lactoferrin opens the iron chelation sites and releases iron ions. In a neutral environment, an apo-lactoferrin can spontaneously re-chelate free iron ions.

The lactoferrin provided in the prior art, such as document 1 (Doi: 10.1016/j.seppur.2020.117522), document 2 (Doi: 10.3168/jds.S0022-0302(95)76862-X.), document 3 (Doi: 10.3168/jds.S0022-0302(84)81345-4.) and patent document CN102459328B, has only two iron ion-binding sites. In addition, as disclosed in document 2, the iron content of Lf is 26 µg/g, iron content is 18%, and therefore, iron saturation content is about 1.4 mg/gprot.

Further, higher iron saturation content of the lactoferrin indicates a stronger iron-absorbing capability, and a faster and stronger iron-absorbing capability enables the lactoferrin to quickly reduce the iron content in a microenvironment, thereby reducing iron to better treat iron overload diseases, and providing a stronger iron-deprivation antibacterial capability. Therefore, it is of great significance to study a preparation method for the lactoferrin having a strong iron-absorbing capability, to further increase the iron-absorbing capability.

Free heme at just 1 µM can cause cell death, exhibiting high toxicity. In many pathological scenarios associated with hemolysis, heme is released due to disruption of blood cells, leading to organ damage. Therefore, it is necessary for proteins to bind to heme, so as to prevent the cells from actively taking up heme, thereby avoiding cell/tissue damage.

Currently, hemopexin (Sigma-Aldrich, H9291) has been approved as a drug for the treatment of sickle cell anemia. Hemopexin is recognized as the strongest known heme-binding protein. Other proteins that can serve as heme-binding proteins include Serum Albumin (Novoprotein, Cat. No.: CP01), Alpha1-Microglobulin (MCE, HY-P7489), Alpha-1-Antitrypsin (Novoprotein, Cat. No.: C533), Lipopolysaccharide-Binding Protein (Novoprotein, Cat. No.: C370), Casein (J&K Scientific, 166199-100G), and transferrin (Thermo Fisher, T8158). Hemopexin has one or two binding sites, Serum Albumin has one binding site, Alpha1-Microglobulin has two binding sites, Alpha-1-Antitrypsin has one binding site, Lipopolysaccharide-Binding Protein has one binding site, Casein has one binding site, and transferrin has four binding sites.

### Technical Problem

However, in the prior art, these heme-binding proteins all have a limited quantity of binding sites, which results in limited heme removal efficiency and requires more proteins to be injected/implanted to absorb a unit amount of heme. Therefore, it is of great significance to study a use of a lactoferrin in binding to heme, to solve the problem in the prior art of the small quantity of heme-binding sites on the proteins.

### Technical Solution

This application provides a lactoferrin having a strong iron-absorbing capability.

This application provides a lactoferrin having a strong iron-absorbing capability. Iron saturation content of the lactoferrin having the strong iron-absorbing capability is above 10 mg/g.

Optionally, the iron saturation content of the lactoferrin having the strong iron-absorbing capability ranges from 10 mg/g to 14.43 mg/g.

Optionally, the lactoferrin having the strong iron-absorbing capability is obtained by methacryloylating a lactoferrin with low iron content.

The lactoferrin with the low iron content exposes a structure within a lactoferrin structure that has a strongest iron-binding capacity, thereby acquiring a strong iron-absorbing capability. After methacryloylation, surface positive charge density of the lactoferrin with the low iron content decreases, facilitating entry of iron ions into the interior of the protein. After methacryloylation, a 3D conformation of the lactoferrin with the low iron content changes (after a chemical structure changes, the protein automatically adjusts its 3D conformation based on the principle of minimum energy), so that more iron-binding sites are generated. Due to increase of the iron-binding sites on the protein, the iron-absorbing capability of the protein is significantly enhanced, and the iron saturation content of the lactoferrin is increased to above 10 mg/g.

Optionally, the lactoferrin with the low iron content refers to a lactoferrin with iron content less than 0.15 mg/g.

Optionally, the iron content of the lactoferrin with the low iron content ranges from 0.0139 mg/g to 0.15 mg/g.

Optionally, the iron content of the lactoferrin with the low iron content ranges from 0.010 mg/g to 0.015 mg/g.

This application further provides a lactoferrin having a strong iron-absorbing capability. The lactoferrin having the strong iron-absorbing capability has 14 to 20 iron ion-binding sites.

This application further provides a lactoferrin. The lactoferrin is a methacryloylated apo-lactoferrin, and the lactoferrin has 7 to 13 heme-binding sites.

The lactoferrin may be the lactoferrin having the strong iron-absorbing capability as described in this application.

Optionally, iron content of the apo-lactoferrin is less than 0.15 mg/g.

Optionally, methacryloylation is performed using methacrylic anhydride, where a grafting ratio of methacrylic anhydride ranges from 30% to 100%.

This application further provides a lactoferrin. A K_{D} value of the lactoferrin binding to heme ranges from 0.015 nM to 2.02 nM.

This application further provides a use of a lactoferrin in binding to heme. That is, the lactoferrin in this application is used for binding to heme.

Heme may form interactions such as hydrogen bonds, salt bridges, and π-π conjugation with amino acids on the lactoferrin. In the methacryloylated lactoferrin, an exposed range of a hydrophobic domain of the protein is increased (a methacryloyl group is a hydrophobic group, so that the hydrophobic domain of the methacryloylated lactoferrin are enlarged), and an electrostatic property is changed (the methacryloyl group is grafted onto lysine, so that a positively charged group is changed to a neutral group, thereby changing the electrostatic property). An increased area of the hydrophobic domain on a protein surface facilitates binding to hydrophobic heme. After amino groups of amino acids are acylated, positive charge on the protein surface decreases, reducing repulsion with positively charged iron at a heme center. This facilitates entry of heme into the interior of the protein, thereby significantly increasing both heme affinity and a heme-binding capacity (that is, the quantity of binding sites).

Optionally, the lactoferrin is prepared as a solution or hydrogel and then injected to bind to free heme.

For example, the lactoferrin is prepared as the solution for intravenous/intraperitoneal/in-situ tissue injection, or prepared as the hydrogel for injectable filling, to bind to free heme. Free heme is free heme generated due to hemolysis in blood as well as free heme generated due to hemolysis in tissue hemorrhage.

Optionally, preparing the lactoferrin as the solution includes:
dissolving the lactoferrin in normal saline to prepare a solution with a concentration ranging from 5 mg/mL to 10 mg/mL.

Optionally, preparing the lactoferrin as the hydrogel includes:
lyophilizing the lactoferrin to obtain lyophilized powder, dissolving the lyophilized powder in a PBS solution to prepare a stock solution, adding an initiator, namely, lithium phenyl-2,4,6-trimethylbenzoylphosphinate, and performing curing under light to form the hydrogel.

Optionally, the initiator is a free radical initiator, a redox initiator, a vitamin C-hydrogen peroxide system, or a glutathione-peroxide system.

Optionally, the free radical initiator is selected from at least one of Irgacure 2959, Irgacure 819, and camphorquinone.

Optionally, the redox initiator includes:
an oxidizing agent, selected from at least one of ammonium persulfate, potassium persulfate, and sodium persulfate; and
a reducing agent, selected from at least one of ascorbic acid, TEMED, sodium sulfite (Na₂SO₃), and sodium bisulfite (NaHSO₃).

Optionally, the vitamin C-hydrogen peroxide system includes:
an oxidizing agent: H₂O₂; and
a reducing agent: ascorbic acid (C₅H₈O₆).

Optionally, the glutathione-peroxide system includes:
an oxidizing agent: urea peroxide (CO(NH₂)₂·H₂O₂); and
a reducing agent: glutathione (GSH, containing a sulfhydryl group).

Optionally, the initiator is a photoinitiator, namely, lithium phenyl-2,4,6-trimethylbenzoylphosphinate, and curing is performed under light to form the hydrogel.

Optionally, in the stock solution, the concentration of the lactoferrin is 0.12 g/mL, and the amount of lithium phenyl-2,4,6-trimethylbenzoylphosphinate is 0.4 wt%.

Optionally, an injection mode is selected from at least one of intravenous injection, intraperitoneal injection, and in-situ tissue injection.

This application further provides a preparation method for a lactoferrin (that is, a lactoferrin having a strong iron-absorbing capability), including: methacryloylating a lactoferrin with low iron content to obtain the lactoferrin having the strong iron-absorbing capability.

Optionally, the method further includes de-ironing a lactoferrin to obtain the lactoferrin with the low iron content.

Optionally, the lactoferrin with the low iron content refers to a lactoferrin with iron content less than 0.15 mg/g.

Optionally, the ratio of the iron content of the lactoferrin with the low iron content to that of the lactoferrin before de-ironing ranges from 3.97% to 9.8%. The ratio of iron content of the lactoferrin having the strong iron-absorbing capability to that of the lactoferrin before de-ironing ranges from 2.86% to 8.5%.

Optionally, the ratio of the iron content of the lactoferrin with the low iron content to that of the lactoferrin before de-ironing is 9.8%. The ratio of iron content of the lactoferrin having the strong iron-absorbing capability to that of the lactoferrin before de-ironing is 8.5%.

Optionally, iron saturation content of the lactoferrin having the strong iron-absorbing capability is above 10 mg/g.

Optionally, the lactoferrin with the low iron content is an apo-lactoferrin.

Optionally, during lactoferrin de-ironing, a lactoferrin solution is first prepared, and after dialysis, retentate is lyophilized to obtain the lactoferrin with the low iron content.

Optionally, during lactoferrin solution dialysis, the method includes placing the lactoferrin solution (a lactoferrin solution to be de-ironed) into a dialysis bag, and placing the dialysis bag in a PBS solution for dialysis for 48 h to 72 h.

Optionally, retentate obtained after dialysis is lyophilized to obtain the apo-lactoferrin.

Optionally, during dialysis, the concentration of the PBS solution in which the dialysis bag is placed is gradually reduced. For example, the PBS dialysis solution may be changed continuously.

The concentration of the PBS solution is gradiently adjusted during a dialysis step of a de-ironing process, so that de-ironing efficiency can be increased. In addition, the lactoferrin with the low iron content is methacryloylated, so that the iron-absorbing capability of the lactoferrin can be enhanced. Iron saturation content is increased to a maximum of 14.43 mg/g, and the quantity of binding sites is increased to a maximum of 20, meaning that the amount of iron ions bound per unit of lactoferrin is increased by 10 times.

Optionally, the concentration of the PBS solution is gradually reduced in the following manner:
from 0 h to 24 h (excluding 24 h), the concentration of the PBS solution is gradually reduced from 0.1 M to 0.05 M;
from 24 h to 48 h (excluding 48 h), the concentration of the PBS solution is gradually reduced from 0.05 M to 0.01 M; and
from 48 h to 72 h, the concentration of the PBS solution is gradually reduced from 0.01 M to 0 M.

By gradually reducing a phosphate concentration in a dialysis environment, that is, through osmotic pressure, iron ions released from the lactoferrin under an acidic environment are continuously removed, thereby increasing the de-ironing efficiency.

Optionally, the lactoferrin solution is prepared by dissolving lactoferrin powder (lactoferrin powder to be de-ironed) in a mixed solution at room temperature.

Optionally, the lactoferrin is a lactoferrin from colostrum, milk, or whey of mammals such as bovines, humans, and camels, as well as a recombinant lactoferrin obtained by fermentation of genetically engineered bacteria or plants.

Optionally, the mixed solution is prepared by adding acetic acid, sodium acetate (a molar ratio of acetic acid to sodium acetate is 1:1), and sodium ethylenediaminetetraacetate (EDTA) to a PBS solution (phosphate buffered saline) with a pH ranging from 2.0 to 4.0. For example, in the mixed solution, the concentration of PBS is 0.01 M, the total concentration of acetic acid and sodium acetate is 0.1 M, and the concentration of EDTA ranges from 40 mM to 500 mM. The pH of the PBS solution used for preparing the mixed solution is adjusted to 2.0 to 4.0 with an acid, and the acid is selected from any one of the following: acetic acid, hydrochloric acid, sulfuric acid, phosphoric acid, or citric acid.

Optionally, the concentration of the lactoferrin solution ranges from 0.5 g/100 mL to 10 g/100 mL.

In the preparation method for the lactoferrin having the strong iron-absorbing capability as described above, the process of methacryloylating the lactoferrin with the low iron content is as follows:
optionally, methacryloylating the lactoferrin with the low iron content using methacrylic anhydride.

Optionally, the process of methacryloylating the lactoferrin with the low iron content includes:
step (1): dissolving the lactoferrin with the low iron content in a PBS solution to prepare a lactoferrin stock solution; and
step (2): adding methacrylic anhydride to the lactoferrin stock solution for reaction to obtain the lactoferrin having the strong iron-absorbing capability.

Optionally, in step (1), the lactoferrin with the low iron content is dissolved in the PBS solution at room temperature to prepare a lactoferrin stock solution with a concentration ranging from 1 g/100 mL to 10 g/100 mL.

Optionally, in step (1), the PBS solution has a pH value ranging from 2.0 to 4.0 (the pH value is, for example, adjusted to 2.0 to 4.0 with an acid selected from any one of the following: acetic acid, hydrochloric acid, sulfuric acid, phosphoric acid, or citric acid).

Optionally, in step (2), a usage ratio of methacrylic anhydride to the lactoferrin with the low iron content ranges from 0.01 mL/1 g to 2 mL/1 g.

Optionally, in step (2), the usage ratio of methacrylic anhydride to the lactoferrin with the low iron content is 0.03 mL/1 g.

Optionally, in step (2), during methacryloylation, a grafting ratio of methacrylic anhydride ranges from 10% to 100%.

Optionally, in step (2), methacrylic anhydride is added to the lactoferrin stock solution under high-speed stirring at 400 to 1,000 rpm.

Optionally, in step (2), after methacrylic anhydride is added to the lactoferrin stock solution, a pH value is adjusted to 6.5 to 7.5 for the reaction.

Optionally, in step (2), after the pH value is adjusted to 6.5 to 7.5, the reaction is carried out under continuous stirring at 10 to 25 °C for 0.5 h to 24 h, and after the reaction, post-treatment is performed to obtain the lactoferrin having the strong iron-absorbing capability.

Optionally, the post-treatment involves dialysis for 24 h to 72 h, concentration by ultrafiltration, then filtration twice through a filtration membrane, and lyophilization of a filtrate to obtain the lactoferrin having the strong iron-absorbing capability.

Optionally, in step (2), the filtration membrane used for the filtration has a pore size of 0.1 µm or 0.22 µm.

This application further provides a formulation for removing heme, and the formulation includes the lactoferrin.

Optionally, the formulation is a microsphere with a porous structure, and the porosity of the formulation is greater than 80%.

The microsphere with the porous structure has a larger specific surface area and a better heme removal effect.

Optionally, the specific surface area of the formulation is greater than 35 m²/g.

Optionally, the formulation has 7 to 13 lactoferrin-binding sites.

This application further provides a preparation method for a formulation for removing heme, including: mixing an aqueous phase and an oil phase to form an emulsion including a photoinitiator, and performing photo-crosslinking on the emulsion to obtain the formulation in the form of a microsphere. In the aqueous phase, water is used as a solvent, and the aqueous phase includes the photoinitiator, the lactoferrin having the strong iron-absorbing capability, and methacryloylated gelatin, and in the oil phase, liquid paraffin is used as a solvent, and the oil phase includes an emulsifier.

Optionally, in the aqueous phase, the content of the lactoferrin ranges from 5% w/v to 30% w/v.

Optionally, in the aqueous phase, the content of methacryloylated gelatin ranges from 1% w/v to 20% w/v.

Optionally, in the aqueous phase, the content of the photoinitiator ranges from 0.1% w/v to 1% w/v.

Optionally, in the aqueous phase, the photoinitiator is lithium phenyl-2,4,6-trimethylbenzoylphosphinate.

Optionally, in the oil phase, the content of the emulsifier ranges from 1% w/v to 20% w/v.

Optionally, in the oil phase, the emulsifier is Span 80.

Optionally, the aqueous phase and the oil phase are each driven by a syringe pump, and the oil phase and the aqueous phase are merged through a channel at a flow rate of 10,000 to 50,000:1 and are emulsified to form droplets.

Optionally, a flow rate of the oil phase is 1 mL/min to 5 mL/min.

Optionally, a flow rate of the aqueous phase ranges from 10 µL/min to 1,000 µL/min.

Optionally, photo-crosslinking is performed on the emulsion under ultraviolet light at 405 nm after the emulsion is frozen.

Optionally, before photo-crosslinking, the emulsion is frozen, where the emulsion is frozen at -100°C to -30°C, and then frozen in liquid nitrogen.

Optionally, before photo-crosslinking, the emulsion is placed at 4 °C for 5 min to 10 min to maintain its morphology.

Optionally, after photo-crosslinking, the obtained microsphere is washed with petroleum ether, acetone at -80°C, and deionized water to obtain the formulation.

This application further provides a use of a formulation in a therapeutic agent for an iron overload disease, where the formulation is the formulation for removing heme according to any one of claims 52 to 55.

Optionally, the iron overload disease is myocardial ischemia-reperfusion injury, renal ischemia-reperfusion injury, or cerebral infarction-reperfusion injury.

Reperfusion therapy is a method for reopening occluded blood vessels through thrombolytic drugs, interventional therapy, or surgical therapy, thereby achieving reperfusion of ischemic tissue. During reperfusion therapy, restoration of blood flow to tissue cells that have suffered ischemia for a specific period may exacerbate structural damage and cause injury, and one of the reasons for this is related to iron overload.

This application further provides a therapy method for an iron overload-related disease, including injecting the formulation described in this application into a lesion site.

Optionally, the iron overload-related disease includes myocardial ischemia-reperfusion injury, renal ischemia-reperfusion injury, or cerebral infarction-reperfusion injury.

Optionally, the formulation is injected into the lesion site using an interventional device including a needle catheter.

This application further provides a reperfusion therapy method, including:
injecting the formulation described in this application into ischemic tissue; and
performing reperfusion therapy on the ischemic tissue.

The formulation described in this application includes a lactoferrin having strong iron-absorbing capability, which can reduce cell damage during reperfusion therapy. A timing of injecting the formulation may be at different stages of reperfusion therapy, and especially before reperfusion therapy. Based on this, it can be understood that this application further provides a method for preventing reperfusion injury, including
optionally, pre-injecting the formulation into the ischemic tissue, and then performing reperfusion therapy.

This application further provides a reperfusion therapy method for myocardial ischemia, including: pre-injecting the formulation described in this application into a left ventricular wall, and then performing reperfusion therapy on the left ventricle.

Optionally, the formulation is injected into the left ventricular wall using an interventional device including a needle catheter, where the needle catheter sequentially passes through a femoral artery and an aortic valve to reach a left ventricular endocardium, and the formulation is injected into the left ventricular wall through the needle catheter.

Optionally, injection points of the needle catheter are distributed at predetermined sites on the left ventricular wall, and there are at least three injection points.

Optionally, an injection volume at each injection point is not less than 0.1 mL.

Optionally, the injection volume at each injection point ranges from 0.1 mL to 0.3 mL.

The lactoferrin having the strong iron-absorbing capability in this application can quickly reduce iron content in a microenvironment, thereby reducing iron to better treat iron overload diseases, which include but are not limited to myocardial ischemia-reperfusion injury, renal ischemia-reperfusion injury, cerebral infarction injury, and other diseases, and providing a stronger iron-deprivation antibacterial capability.

### Beneficial Effects

In this application, lactoferrin is modified with a reasonable methacrylic anhydride grafting degree, which can enhance a heme-absorbing capacity of the lactoferrin. The methacryloylated lactoferrin has an increased quantity of heme-binding pockets, and its affinity and binding capacity for heme are significantly enhanced.

### Brief Description of the Drawings

FIG. 1 is an ICP test chart of a lactoferrin raw material, an apo-lactoferrin (Apo-Lf), and a lactoferrin having a strong iron-absorbing capability (Apo-LfMA) in Embodiment 1.
FIG. 2 is a nuclear magnetic resonance spectrum of an apo-lactoferrin (Apo-Lf) and a lactoferrin having a strong iron-absorbing capability (Apo-LfMA) in Embodiment 1.
FIG. 3 is a MALDI-TOF diagram of an apo-lactoferrin (Apo-Lf) and a lactoferrin having a strong iron-absorbing capability (Apo-LfMA) in Embodiment 1.
FIG. 4 is a diagram of a 3D structural change from an apo-lactoferrin (Apo-Lf) to an obtained lactoferrin having a strong iron-absorbing capability (Apo-LfMA) before and after methacrylic anhydride modification in Embodiment 1.
FIG. 5 is a schematic diagram of specific amino acid sites on an Apo-Lf modified with methacrylic anhydride.
FIG. 6 is a molecular docking diagram of a lactoferrin having a strong iron-absorbing capability (Apo-LfMA) binding to iron ions, where spheres represent the iron ions, indicating that the protein chelates 20 iron ions.
FIG. 7 to FIG. 16 are diagrams of 20 specific iron chelation sites of an Apo-LfMA obtained through methacrylic anhydride grafting, that is, amino acid composition and iron chelation interaction analysis.
FIG. 17 is a comparison diagram of iron saturation content of an apo-lactoferrin (Apo-Lf) and iron saturation content of a lactoferrin having a strong iron-absorbing capability (Apo-LfMA) in Embodiment 1.
FIG. 18 is an ICP test chart of an apo-lactoferrin (Apo-Lf) at pH=4.0 in Embodiment 2 and at pH=2.0 in Embodiment 1.
FIG. 19 shows MALDI-TOF with a methacrylic anhydride grafting degree of 10% (10% Apo-LfMA) in Embodiment 3 (left side of the figure) and a MALDI-TOF with a methacrylic anhydride grafting degree of 100% (100% Apo-LfMA) in Embodiment 4 (right side of the figure).
FIG. 20 shows iron saturation content with a methacrylic anhydride grafting degree of 10% (10% Apo-LfMA) in Embodiment 3 and iron saturation content with a methacrylic anhydride grafting degree of 100% (100% Apo-LfMA) in Embodiment 4.
FIG. 21 is an ICP test chart of a lactoferrin raw material, an apo-lactoferrin (Apo-Lf), and a methacryloylated lactoferrin (Apo-LfMA) in Embodiment 5, showing iron content of raw materials.
FIG. 22 is a diagram of a 3D structural change from an apo-lactoferrin (Apo-Lf) to a methacryloylated lactoferrin (Apo-LfMA) before and after methacrylic anhydride modification in Embodiment 5.
FIG. 23 is a schematic diagram of specific amino acid sites on an Apo-Lf modified with methacrylic anhydride.
FIG. 24 shows a change in protein surface electrostatic property from an apo-lactoferrin (Apo-Lf) to a methacryloylated lactoferrin (Apo-LfMA) before and after methacrylic anhydride modification in Embodiment 5.
FIG. 25 shows a change in protein surface hydrophobicity from an apo-lactoferrin (Apo-Lf) to a methacryloylated lactoferrin (Apo-LfMA) before and after methacrylic anhydride modification in Embodiment 5, where a and b represent the hydrophobicity of two sides of the protein, respectively.
FIG. 26 shows molecular dynamics simulation images of binding of an apo-lactoferrin (Apo-Lf) to heme and binding of a methacryloylated lactoferrin (Apo-LfMA) to heme in Embodiment 5, where Site1, Site2, ... indicate the heme-binding sites on the protein.
FIG. 27 shows surface plasmon resonance (SPR) spectra of binding of an apo-lactoferrin (Apo-Lf) to heme and binding of a methacryloylated lactoferrin (Apo-LfMA) to heme in Embodiment 5, where Raw represents a raw curve and Fitting represents a fitting curve.
FIG. 28 shows comparison between the amount of heme bound to an apo-lactoferrin (Apo-Lf) and the amount of heme bound to a methacryloylated lactoferrin (Apo-LfMA) in Embodiment 5.
FIG. 29 is an ICP test chart of an apo-lactoferrin obtained through de-ironing (Apo-LfMA) at pH=4.0 in Embodiment 6 and at pH=2.0 in Embodiment 5.
FIG. 30 shows surface plasmon resonance (SPR) spectra of binding of heme to a methacryloylated lactoferrin (Apo-LfMA) obtained from an apo-lactoferrin (Apo-Lf) obtained through de-ironing at pH=4.0 in Embodiment 6 and at pH=2.0 in Embodiment 5, indicating constants of the protein binding to heme, where Raw represents a raw curve and Fitting represents a fitting curve.
FIG. 31 shows the amounts of heme bound to a methacryloylated lactoferrin (Apo-LfMA) obtained from an apo-lactoferrin (Apo-Lf) obtained through de-ironing at pH=4.0 in Embodiment 6 and at pH=2.0 in Embodiment 5.
FIG. 32 shows a surface plasmon resonance (SPR) spectrum of binding of heme to methacryloylated apo-lactoferrin (100% Apo-LfMA) in Embodiment 7, where Raw represents a raw curve and Fitting represents a fitting curve.
FIG. 33 shows the amount of heme bound to a methacryloylated apo-lactoferrin (100% Apo-LfMA) in Embodiment 7.
FIG. 34 is a bar chart of the specific surface areas of ALMG and ALMS.
FIG. 35 is a bar chart of the porosity of ALMG and ALMS.
FIG. 36 is a bar chart of numbers of saturated heme bound to ALMG and ALMS.
FIG. 37 shows live/dead staining images and intracellular ferrous ion staining images of cardiomyocytes treated with heme for 24 h.
FIG. 38 is a cell viability chart of cardiomyocytes treated with heme for 24 h in the presence of GMG, ALMG or ALMS (five bars in the figure from left to right correspond to Control, Haem, Haem+GMG, Haem+ALMG, Haem+ALMS, respectively).
FIG. 39 is an average fluorescence intensity chart of intracellular ferrous ions (Fe²⁺) in cardiomyocytes treated with heme for 24 h in the presence of GMG, ALMG or ALMS (five bars in the figure from left to right correspond to Control, Haem, Haem+GMG, Haem+ALMG, Haem+ALMS, respectively).
FIG. 40 shows TEM images of the heart of an I/R rat with an IMH phenotype.
FIG. 41 is a heme concentration chart after injection of different substances (four bars in the figure from left to right correspond to Sham, I/R, GMG, and ALMS, respectively). FIG. 42 is a myocardial iron content chart after injection of different substances (four bars in the figure from left to right correspond to Sham, I/R, GMG, and ALMS, respectively).
FIG. 43 shows immunofluorescence staining images of HBB and Hmox1.
FIG. 44 shows immunofluorescence staining images of HBB and TUNEL, where scale bar=20 µm (there are three lines in each of the upper and lower images, and three lines in each group from top to bottom correspond to HBB, TUNEL, and DAPI, respectively).
FIG. 45 shows DAB-Perls' blue and 4-HNE (4-hydroxynonenal) staining images of rat heart sections.
FIG. 46 shows charts of levels of myocardial injury markers in an ALMS group (there are four charts a, b, c, and d, each including four bars, and the bars from left to right correspond to Sham, I/R, GMG, and ALMS, respectively).
FIG. 47 shows ultrasound images of an infarct size and a left ventricular wall thickness under different treatment conditions.
FIG. 48 shows value charts of left ventricular ejection fractions (LVEFs) and left ventricular fractional shortening (LVFS) under different treatment conditions (each chart includes four bars, and the bars from left to right correspond to Sham, I/R, GMG, and ALMS, respectively).
FIG. 49 shows masson staining images under different treatment conditions.
FIG. 50 shows infarct sizes and left ventricular wall thicknesses under different treatment conditions (each chart includes four bars, and the bars from left to right correspond to Sham, I/R, GMG, and ALMS, respectively).
FIG. 51 is a schematic diagram of a path for injecting ALMS into an inner wall of a left ventricle using a transcatheter system.
FIG. 52 is a schematic diagram of TTC staining of left ventricular tissue.
FIG. 53 is a comparison diagram of necrotic myocardial volumes under different treatment conditions (which correspond, in the figure, from left to right, to Sham, I/R, and ALMS, respectively).
FIG. 54 is a comparison diagram of creatine kinase isoenzyme (CK-MB) under different treatment conditions (which correspond, in the figure, from left to right, to Sham, I/R, and ALMS, respectively).
FIG. 55 is a comparison diagram of creatine kinase (CK) under different treatment conditions (which correspond, in the figure, from left to right, to Sham, I/R, and ALMS, respectively).
FIG. 56 is a comparison diagram of lactate dehydrogenase (LDH) under different treatment conditions (which correspond, in the figure, from left to right, to Sham, I/R, and ALMS, respectively).
FIG. 57 is a comparison diagram of aspartate aminotransferase (AST) under different treatment conditions (which correspond, in the figure, from left to right, to Sham, I/R, and ALMS, respectively).
FIG. 58 is a comparison diagram of cardiac troponin I (cTnI) under different treatment conditions (which correspond, in the figure, from left to right, to Sham, I/R, and ALMS, respectively).
FIG. 59 is a comparison diagram of cardiac heme under different treatment conditions (which correspond, in the figure, from left to right, to Sham, I/R, and ALMS, respectively).
FIG. 60 is a comparison diagram of heme oxygenase 1 (HMOX1) under different treatment conditions (which correspond, in the figure, from left to right, to Sham, I/R, and ALMS, respectively).
FIG. 61 shows hematoxylin and eosin (H&E) staining images of tissue sections of pig liver, spleen, lung, and kidney (n=3).

### Detailed Description

The present invention is further described below with reference to specific implementations. It should be understood that these embodiments are for illustrative purposes only and are not intended to limit the scope of the present invention. Furthermore, it should be understood that after reading content described in the present invention, those skilled in the art can make various alterations or modifications to the present invention, and these equivalent forms also fall within the scope defined by the claims appended to this application.

Test methods involved in the present invention are as follows.

Iron content: 1 mL of concentrated nitric acid is added to 5 mg of protein powder, digestion is carried out at 70 °C for 1 h, after cooling to room temperature, dilution is carried out with deionized water to 5 mL, and a solution obtained after dilution is submitted to inductively coupled plasma (ICP) to test an iron ion concentration, where an obtained concentration value a µg/mL is equal to iron content a mg/g in a protein.

Iron saturation content: 100 mg of a protein is dissolved in 50 mL of a FeCl₃/NaHCO₃ (at a molar ratio of 1:1) solution with a concentration of 50 µg/mL and a pH ranging from 7.2 to 7.4, stirring is carried out for 30 min, a mixed solution is centrifuged in a 50 kDa ultrafiltration tube to remove unbound excess iron ions, the protein is washed three times with deionized water until all unbound iron ions are removed, and the protein is then lyophilized into powder. 1 mL of concentrated nitric acid is added to 5 mg of the lyophilized protein powder, digestion is carried out at 70 °C for 1 h, after cooling to room temperature, dilution is carried out with deionized water to 5 mL, and a solution obtained after dilution is submitted to inductively coupled plasma (ICP) to test an iron ion concentration, where an obtained concentration value a µg/mL is equal to iron saturation content a mg/g in the protein.

Value of a binding constant K_{D}: The technique used is a surface plasmon resonance (SPR) technique with a Biacore instrument. The SPR principle is used to detect an interaction between a ligand on a biosensor chip and an analyte. To study an interaction between two molecules, one molecule is immobilized onto a chip surface, while the other molecule flows continuously over the chip surface in the form of a solution. A detector detects association and dissociation processes between the molecule in the solution and the molecule on the chip surface in real time, and the processes are displayed in the form of SPR response values. Calculation formula: K_{D}=kd/ka, where ka is an affinity rate at which heme is bound to the protein, and kd is a dissociation rate.

### Embodiment 1

A preparation method for a lactoferrin having a strong iron-absorbing capability is provided, and specific steps are as follows.
(1) Acetic acid, sodium acetate (at a molar ratio of 1:1), and EDTA are added to a PBS solution with a pH of 2.0 to prepare a mixed solution, and lactoferrin powder (Bega, Australia) is then dissolved in the mixed solution at room temperature to prepare a lactoferrin solution with a concentration of 0.5 g/100 mL.
   In the mixed solution, the concentration of PBS is 0.01 M, the total concentration of acetic acid and sodium acetate is 0.1 M, and the concentration of EDTA is 500 mM.
(2) The lactoferrin solution obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.0139 mg/g. During dialysis, the PBS solution is continuously replaced, and the concentration of the PBS solution is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.
   A molecular weight cutoff of the dialysis bag is 3,500 Da.
(3) The apo-lactoferrin obtained in step (2) is methacryloylated to obtain a lactoferrin having a strong iron-absorbing capability.
(3.1) The apo-lactoferrin is dissolved in a PBS solution with a pH of 2 at room temperature to prepare an apo-lactoferrin stock solution with a concentration of 10 g/100 mL.
(3.2) Under high-speed stirring at 600 rpm, methacrylic anhydride is added to the apo-lactoferrin stock solution, the pH value is then adjusted to 7, reaction is carried out under continuous stirring at 25 °C for 2 h, after the reaction, dialysis is carried out for 48 h, after concentration by ultrafiltration, filtering is carried out twice through a filtration membrane with a pore size of 0.22 µm, and a filtrate is first pre-frozen at a temperature of -50°C and then lyophilized under a vacuum of 1 Pa to obtain a lactoferrin having a strong iron-absorbing capability with ferric iron saturation content of 14.43 mg/g.

A usage ratio of methacrylic anhydride to the lactoferrin is 0.03 mL/1 g, and a grafting degree of methacrylic anhydride on the lactoferrin having the strong iron-absorbing capability is 30%.

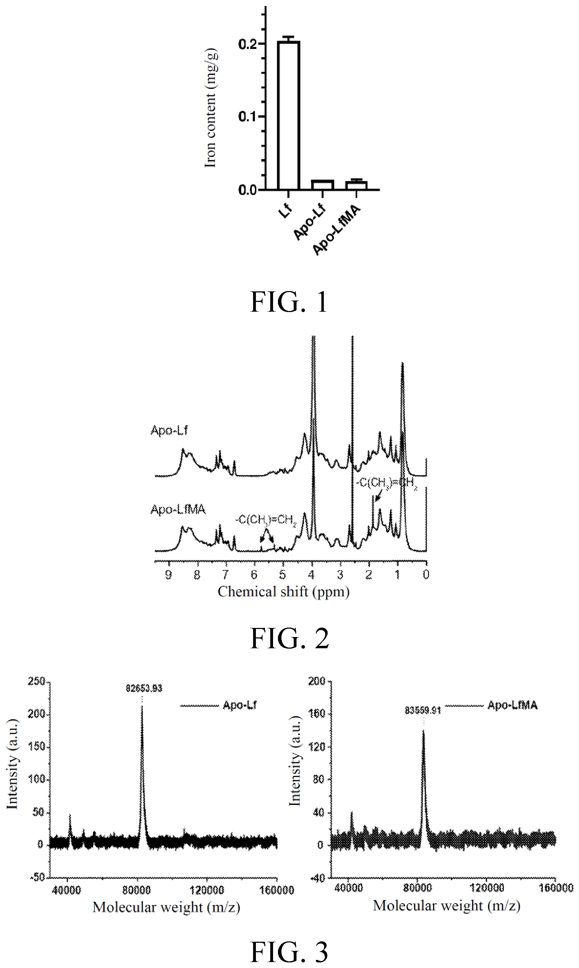

As shown in FIG. 1, through de-ironing, an Apo-Lf and an Apo-LfMA are obtained with iron content of 9.8% and iron content of 8.5% of that of Lf, respectively.

As shown in FIG. 2 and FIG. 3, methacrylic anhydride is successfully grafted onto the Apo-Lf with reactive double bonds, so that a molecular weight of the methacryloylated Apo-LfMA is increased.

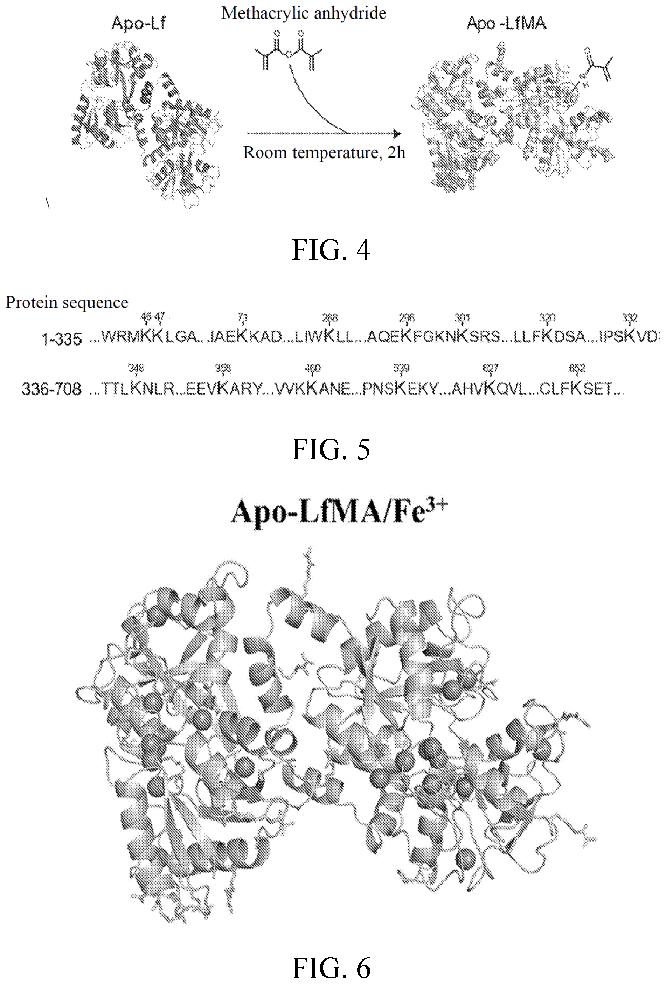

FIG. 4 shows a 3D structural change from the Apo-Lf to the Apo-LfMA before and after methacrylic anhydride grafting (protein morphology is constructed by using AlphaFold).

FIG. 5 is a schematic diagram of specific amino acid (lysine) sites on the Apo-Lf modified by methacrylic anhydride, and 14 specific sites are shown in Table 1 below. Lowercase k indicates modified positions of specific lysine, and a new Apo-LfMA protein structure is obtained for subsequent molecular docking analysis of iron ions.

It can be seen from FIG. 4 and FIG. 6 that after methacryloylation, a 3D conformation of a lactoferrin with low iron content changes (after a chemical structure changes, the protein automatically adjusts its 3D conformation based on the principle of minimum energy), so that more iron-binding sites are generated.

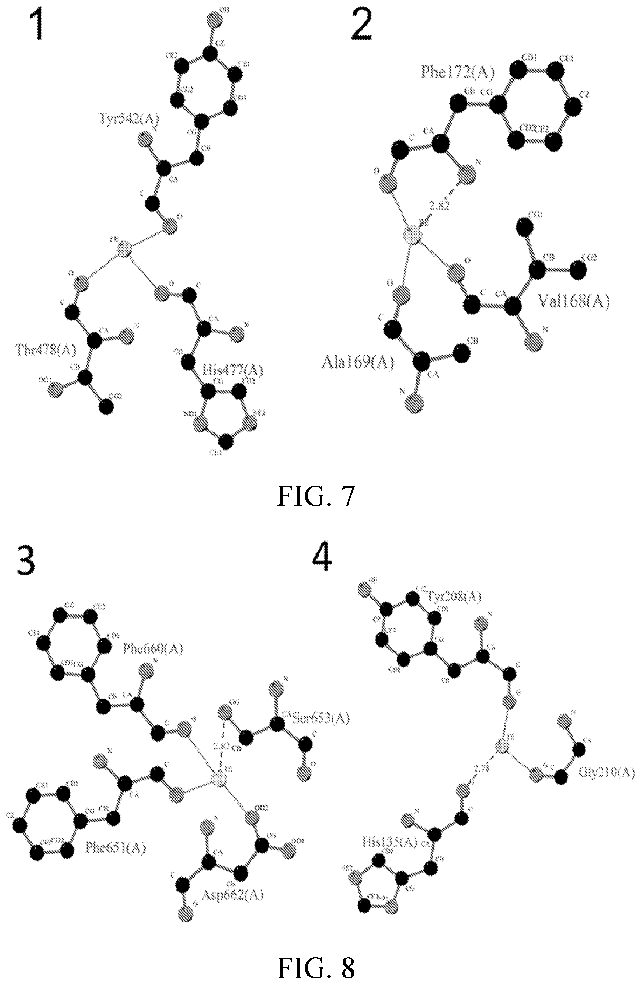

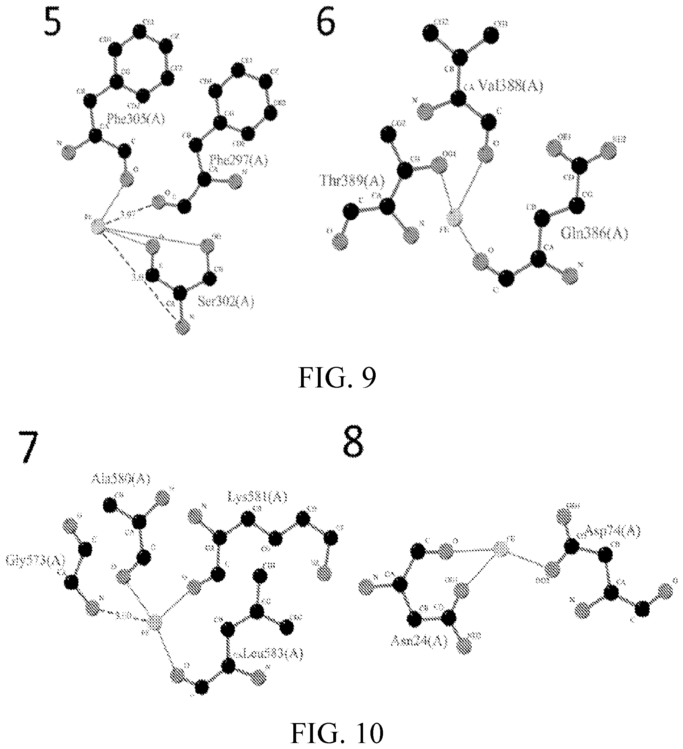

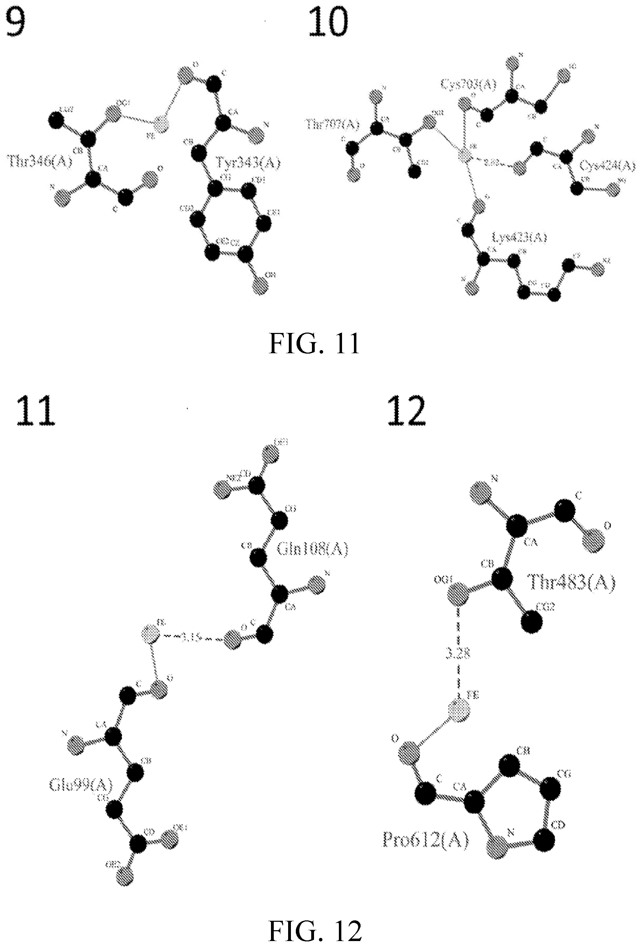

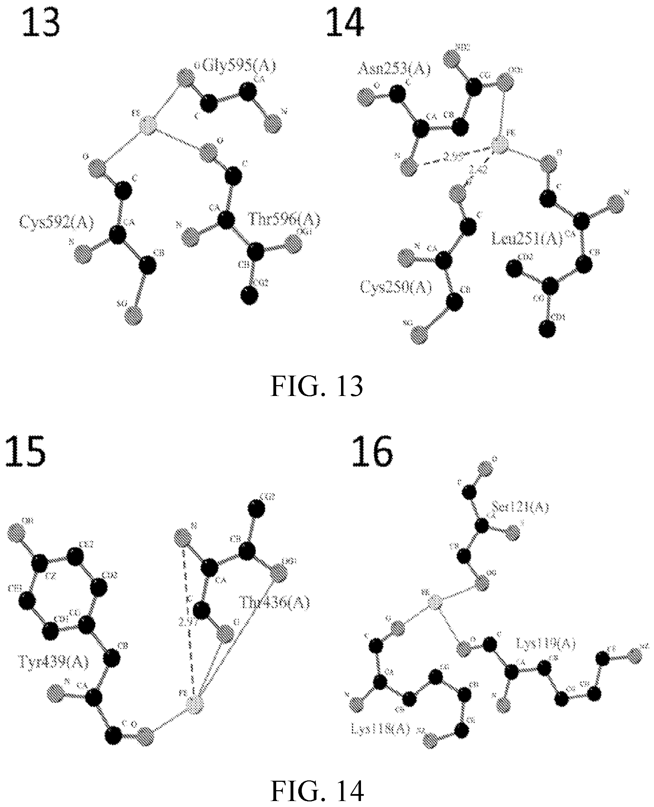

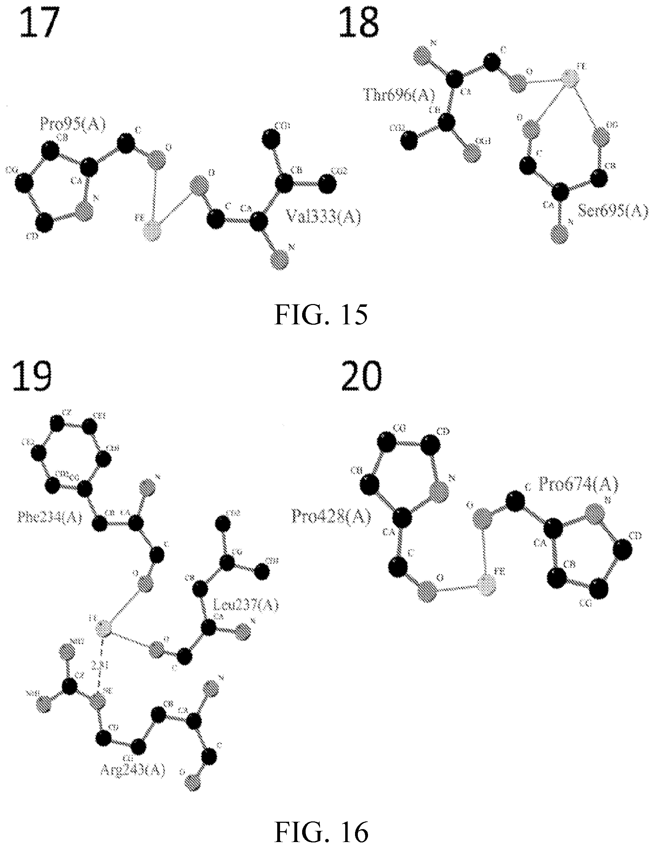

Through molecular docking of the Apo-LfMA with the iron ions, a total of 20 iron chelation sites of the Apo-LfMA obtained through methacrylic anhydride grafting are obtained. Detailed analysis diagrams of the 20 sites are shown in FIG. 7 to FIG. 16.

**Table 1**

| | |
|---|---|
| K46 | FKCRRWQWRMkKLGAPSITCV |
| K47 | KCRRWQWRMKkLGAPSITCVR |
| K71 | ALECIRAIAEkKADAVTLDGG |
| K288 | SVDGKEDLIWkLLSKAQEKFG |
| K296 | IWKLLSKAQEkFGKNKSRSFQ |
| K301 | SKAQEKFGKNkSRSFQLFGSP |
| K320 | SPPGQRDLLFkDSALGFLRIP |
| K332 | SALGFLRIPSkVDSALYLGSR |
| K348 | YLGSRYLTTLkNLRETAEEVK |
| K358 | KNLRETAEEVkARYTRVVWCA |
| K460 | EGYLAVAVVKkANEGLTWNSL |
| K539 | QGLDKCVPNSkEKYYGYTGAF |
| K627 | VSRSDRAAHVkQVLLHQQALF |
| K652 | KNCPDKFCLFkSETKNLLFND |

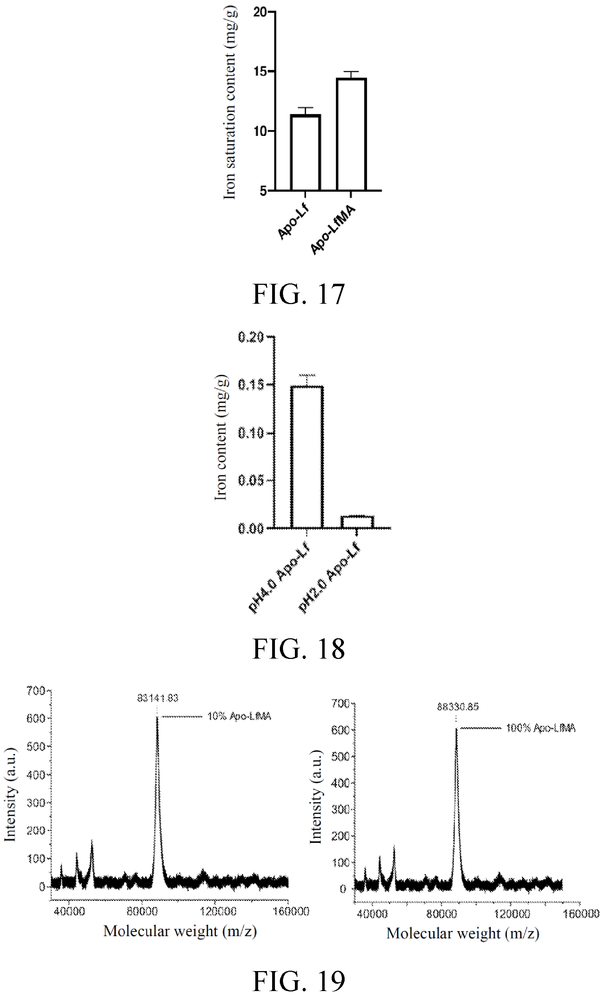

As shown in FIG. 17, the saturation content of ferric iron binding to the methacryloylated lactoferrin is significantly increased.

### Embodiment 2+-

A preparation method for a lactoferrin having a strong iron-absorbing capability is provided, and specific steps are as follows.
(1) Acetic acid, sodium acetate (at a molar ratio of 1:1), and EDTA are added to a PBS solution with a pH of 4.0 to prepare a mixed solution, and lactoferrin powder (Bega, Australia) is then dissolved in the mixed solution at room temperature to prepare a lactoferrin solution with a concentration of 10g/100 mL.
   In the mixed solution, the concentration of PBS is 0.01 M, the total concentration of acetic acid and sodium acetate is 0.1 M, and the concentration of EDTA is 40 mM.
(2) The lactoferrin solution obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.149 mg/g. During dialysis, the PBS solution is continuously replaced, and the concentration of the PBS solution is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.

A molecular weight cutoff of the dialysis bag is 7,000 Da.

As shown in FIG. 18, in Embodiment 2, a pH for de-ironing is increased to 4.0, thereby reducing de-ironing efficiency, and iron content of the Apo-Lf obtained through de-ironing at pH=4.0 is 0.149 mg/g, which is 10 times higher than that at pH=2.0 in Embodiment 1.
(3) The apo-lactoferrin obtained in step (2) is methacryloylated to obtain a lactoferrin having a strong iron-absorbing capability.
(3.1) The apo-lactoferrin is dissolved in a PBS solution with a pH of 4 at room temperature to prepare an apo-lactoferrin stock solution with a concentration of 10 g/100 mL.
(3.2) Under high-speed stirring at 600 rpm, methacrylic anhydride is added to the apo-lactoferrin stock solution, the pH value is then adjusted to 7.2, reaction is carried out under continuous stirring at 25 °C for 2 h, after the reaction, dialysis is carried out for 48 h, after concentration by ultrafiltration, filtering is carried out twice through a filtration membrane with a pore size of 0.22 µm, and a filtrate is first pre-frozen at a temperature of -50°C and then lyophilized under a vacuum of 1 Pa to obtain a lactoferrin having a strong iron-absorbing capability with ferric iron saturation content of 14.43 mg/g.

A usage ratio of methacrylic anhydride to the lactoferrin is 0.03 mL/1 g, and a grafting degree of methacrylic anhydride on the lactoferrin having the strong iron-absorbing capability is 30%.

### Embodiment 3

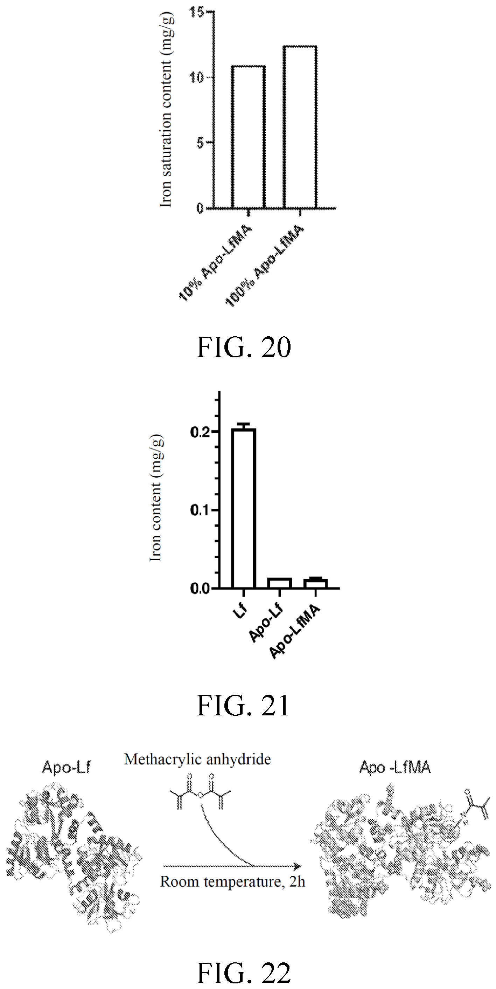

A preparation method for a lactoferrin having a strong iron-absorbing capability is provided, and specific steps are as follows.
(1) Acetic acid, sodium acetate (at a molar ratio of 1:1), and EDTA are added to a PBS solution to prepare a mixed solution, and lactoferrin powder (Bega, Australia) is then dissolved in the mixed solution at room temperature to prepare a lactoferrin solution with a concentration of 0.5 g/100 mL.
   In the mixed solution, the concentration of PBS is 0.01 M, the total concentration of acetic acid and sodium acetate is 0.1 M, and the concentration of EDTA is 500 mM.
(2) The lactoferrin solution obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.0139 mg/g. During dialysis, the PBS solution is continuously replaced, and the concentration of the PBS solution is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.
   A molecular weight cutoff of the dialysis bag is 7,000 Da.
(3) The apo-lactoferrin obtained in step (2) is methacryloylated to obtain a lactoferrin having a strong iron-absorbing capability.
(3.1) The apo-lactoferrin is dissolved in a PBS solution with a pH of 2 at room temperature to prepare an apo-lactoferrin stock solution with a concentration of 10 g/100 mL.
(3.2) Under high-speed stirring at 400 rpm, methacrylic anhydride is added to the apo-lactoferrin stock solution, the pH value is then adjusted to 7.5, reaction is carried out under continuous stirring at 20 °C for 5 h, after the reaction, dialysis is carried out for 48 h, after concentration by ultrafiltration, filtering is carried out twice through a filtration membrane with a pore size of 0.22 µm, and a filtrate is first pre-frozen at a temperature of -50°C and then lyophilized under a vacuum of 1 Pa to obtain a lactoferrin having a strong iron-absorbing capability with ferric iron saturation content of 10.95 mg/g as shown in FIG. 19 and FIG. 20.

A usage ratio of methacrylic anhydride to the lactoferrin is 0.01 mL/1 g, and a grafting degree of methacrylic anhydride on the lactoferrin having the strong iron-absorbing capability is 10%.

### Embodiment 4

A preparation method for a lactoferrin having a strong iron-absorbing capability is provided, and specific steps are as follows.
(1) Acetic acid, sodium acetate (at a molar ratio of 1:1), and EDTA are added to a PBS solution to prepare a mixed solution, and lactoferrin powder (Bega, Australia) is then dissolved in the mixed solution at room temperature to prepare a lactoferrin solution with a concentration of 10g/100 mL.
   In the mixed solution, the concentration of PBS is 0.01 M, the total concentration of acetic acid and sodium acetate is 0.1 M, and the concentration of EDTA is 500 mM.
(2) The lactoferrin solution obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.0139 mg/g. During dialysis, the PBS solution is continuously replaced, and the concentration of the PBS solution is gradually reduced. From 0 h to 24 h, the concentration of the PBS solution is 0.1 M; from 24 h to 48 h, the concentration of the PBS solution is 0.05 M; and from 48 h to 72 h, the concentration of the PBS solution is 0 M.
   A molecular weight cutoff of the dialysis bag is 7,000 Da.
(3) The apo-lactoferrin obtained in step (2) is methacryloylated to obtain a lactoferrin having a strong iron-absorbing capability.
(3.1) The apo-lactoferrin is dissolved in a PBS solution with a pH of 2 at room temperature to prepare an apo-lactoferrin stock solution with a concentration of 1 g/100 mL.
(3.2) Under high-speed stirring at 1,000 rpm, methacrylic anhydride is added to the apo-lactoferrin stock solution, the pH value is then adjusted to 6.5, reaction is carried out under continuous stirring at 10 °C for 24 h, after the reaction, dialysis is performed for 72 h, after concentration by ultrafiltration, filtering is carried out twice through a filtration membrane with a pore size of 0.22 µm, and a filtrate is first pre-frozen at a temperature of -50°C and then lyophilized under a vacuum of 1 Pa to obtain a lactoferrin having a strong iron-absorbing capability with ferric iron saturation content of 12.46 mg/g as shown in FIG. 19 and FIG. 20.

A usage ratio of methacrylic anhydride to the lactoferrin is 2 mL/1 g, and a grafting degree of methacrylic anhydride on the lactoferrin having the strong iron-absorbing capability is 100%.

### Embodiment 5

A use of a lactoferrin in binding to heme is provided, and specific steps are as follows.
(1) Acetic acid, sodium acetate (at a molar ratio of 1:1), and EDTA are added to a PBS solution with a pH of 2.0 to prepare a mixed solution. Lactoferrin powder to be de-ironed (Bega, Australia) is then dissolved in the mixed solution at room temperature to prepare a lactoferrin solution to be de-ironed with a concentration of 10 g/100 mL.
   In the mixed solution, the concentration of PBS is 0.01 M, the total concentration of acetic acid and sodium acetate is 0.1 M, and the concentration of EDTA is 500 mM.
(2) The lactoferrin solution to be de-ironed obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.0139 mg/g.
   A molecular weight cutoff of the dialysis bag is 7,000 Da. The concentration of the PBS solution for dialysis is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.
(3) The apo-lactoferrin obtained in step (2) is dissolved in a PBS solution with a pH of 2.0 at room temperature to prepare an apo-lactoferrin stock solution with a concentration of 10 g/100 mL.
(4) Under high-speed stirring at 400 rpm, methacrylic anhydride is added to the apo-lactoferrin stock solution, the pH is then adjusted to 7, reaction is carried out under continuous stirring at 25 °C for 2 h, after the reaction, dialysis is performed for 72 h, after concentration by ultrafiltration, filtering is carried out twice through a filtration membrane with a pore size of 0.22 µm, and a filtrate is lyophilized at a temperature of -50°C under a vacuum of 1 Pa to obtain a methacryloylated apo-lactoferrin.

A usage ratio of methacrylic anhydride to the apo-lactoferrin is 0.03 mL/1 g. A grafting degree of methacrylic anhydride on the methacryloylated apo-lactoferrin is 30%.

The prepared methacryloylated lactoferrin is dissolved in normal saline to prepare a solution with a concentration of 5 mg/mL, which is then injected for binding to free heme.

The methacryloylated lactoferrin has 13 heme-binding sites. A K_{D} value of the lactoferrin binding to heme is 0.015 mM.

As shown in FIG. 21, through de-ironing, an Apo-Lf and an Apo-LfMA with iron content of 9.8% and iron content of 8.5% of that of Lf, respectively, are obtained.

FIG. 22 shows a 3D structural change from the Apo-Lf to the Apo-LfMA before and after methacrylic anhydride grafting (protein morphology is constructed by using AlphaFold).

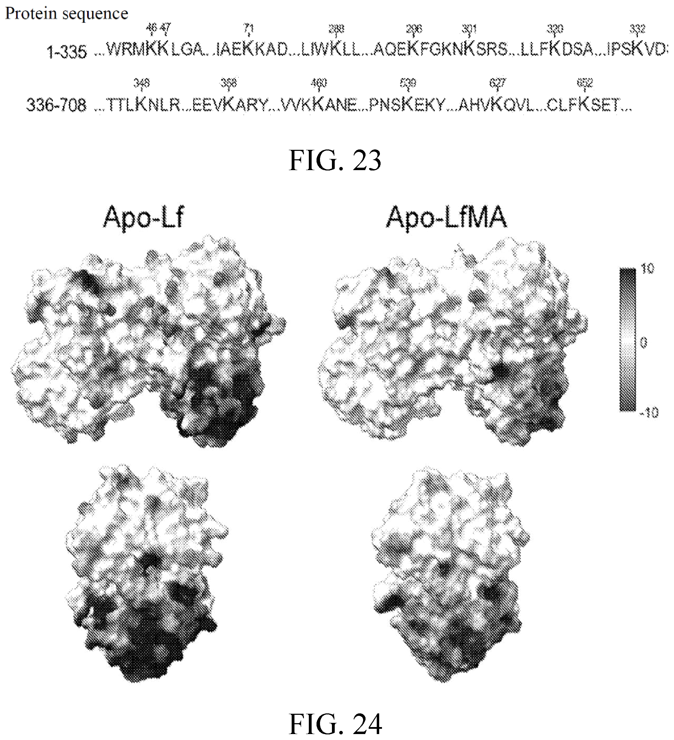

FIG. 23 is a schematic diagram of specific amino acid sites on an Apo-Lf modified with methacrylic anhydride.

As shown in FIG. 24, surface positive charge density of a methacryloylated lactoferrin with a grafting degree of 30% decreases, and the resistance for a positively charged iron ion at a heme center to enter the interior of the protein is reduced.

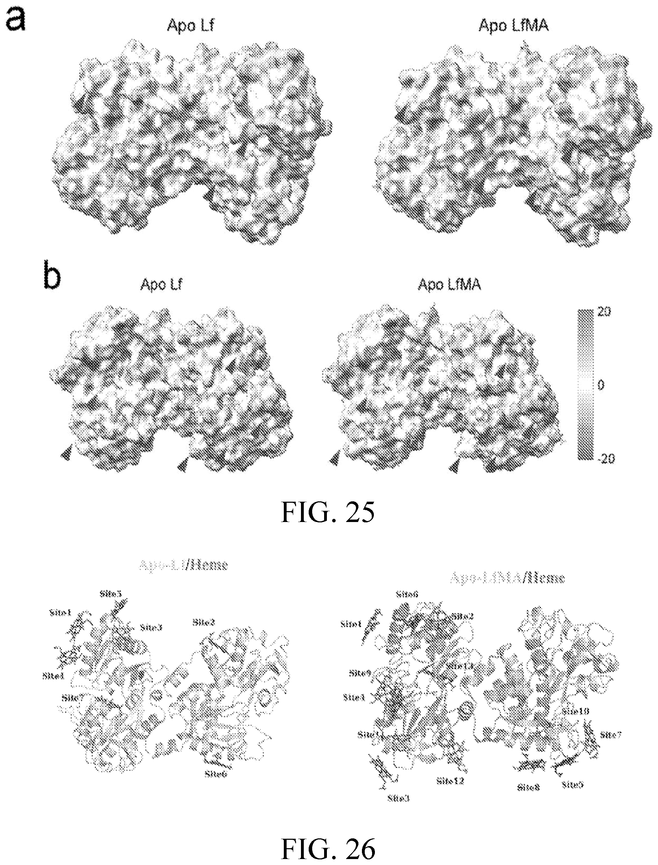

As shown in FIG. 25, an increased exposed area of a hydrophobic domain on a surface of the methacryloylated lactoferrin with the grafting degree of 30% facilitates binding to hydrophobic heme.

As shown in FIG. 26, the amount of heme bound to the methacryloylated lactoferrin with the grafting degree of 30% is higher than that bound to an ungrafted lactoferrin.

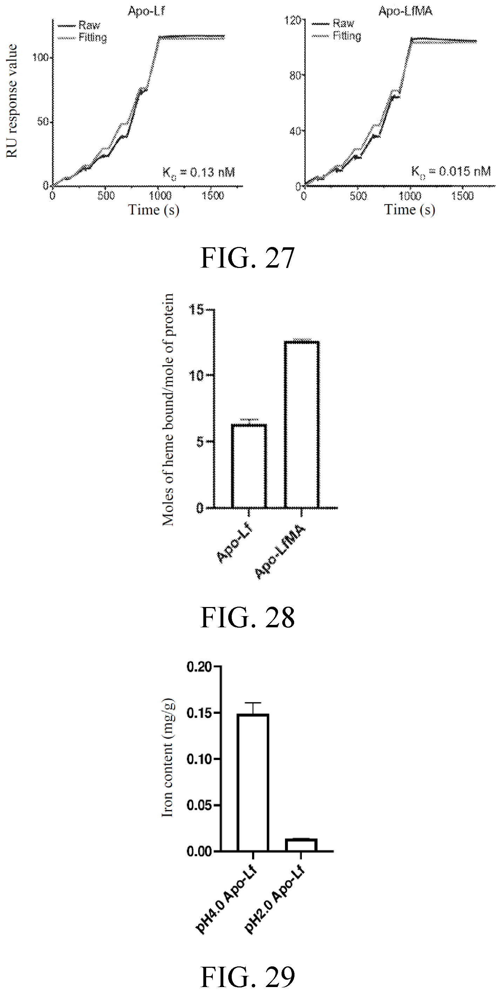

As shown in FIG. 27, saturation content of heme bound to the methacryloylated lactoferrin with the grafting degree of 30%, and its affinity increases (which is reflected by a decrease of a K_{D} value).

As shown in FIG. 28, the amount of heme bound to the methacryloylated lactoferrin increases.

### Embodiment 6

A use of a lactoferrin in binding to heme is provided, and specific steps are as follows.
(1) Acetic acid, sodium acetate (at a molar ratio of 1:1), and EDTA are added to a PBS solution with a pH of 4.0 to prepare a mixed solution, and lactoferrin powder to be de-ironed (Bega, Australia) is then dissolved in the mixed solution at room temperature to prepare a lactoferrin solution to be de-ironed with a concentration of 10 g/100 mL.
In the mixed solution, the concentration of PBS is 0.01 M, the total concentration of acetic acid and sodium acetate is 0.1 M, and the concentration of EDTA is 500 mM.

### (2) Preparation of an apo-lactoferrin

The lactoferrin solution to be de-ironed obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.149 mg/g.

A molecular weight cutoff of the dialysis bag is 7,000 Da. The concentration of the PBS solution for dialysis is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.

As shown in FIG. 29, a pH for de-ironing is increased to 4.0, thereby reducing de-ironing efficiency, and iron content of the Apo-Lf obtained through de-ironing at pH=4.0 is 0.149 mg/g, which is 10 times higher than that at pH=2.0.
(3) The apo-lactoferrin obtained in step (2) is dissolved in a PBS solution with a pH of 4.0 at room temperature to prepare an apo-lactoferrin stock solution with a concentration of 10 g/100 mL.
(4) Under high-speed stirring at 40 rpm, methacrylic anhydride is added to the apo-lactoferrin stock solution, the pH is then adjusted to 7, reaction is carried out under continuous stirring at 25 °C for 2 h, after the reaction, dialysis is performed for 72 h, after concentration by ultrafiltration, filtering is carried twice through a filtration membrane with a pore size of 0.22 µm, and a filtrate is lyophilized at a temperature of -50°C under a vacuum of 1 Pa to obtain a methacryloylated apo-lactoferrin.

A usage ratio of methacrylic anhydride to the apo-lactoferrin is 0.03 mL/1 g. A grafting degree of methacrylic anhydride on the methacryloylated apo-lactoferrin is 30%.

Lyophilized powder obtained by lyophilizing the prepared methacryloylated apo-lactoferrin is dissolved in a 0.2 M PBS solution to prepare a 0.12 g/mL stock solution. Then, 0.4 wt% lithium phenyl-2,4,6-trimethylbenzoylphosphinate is added, and stirring is carried out for dissolving. Sterile filtration is carried out to obtain an aqueous phase solution, and the solution is cured under 405 nm light to form a hydrogel, which is then injected for binding to free heme.

The methacryloylated lactoferrin has 11 heme-binding sites. A K_{D} value of the lactoferrin binding to heme is 0.38 nM.

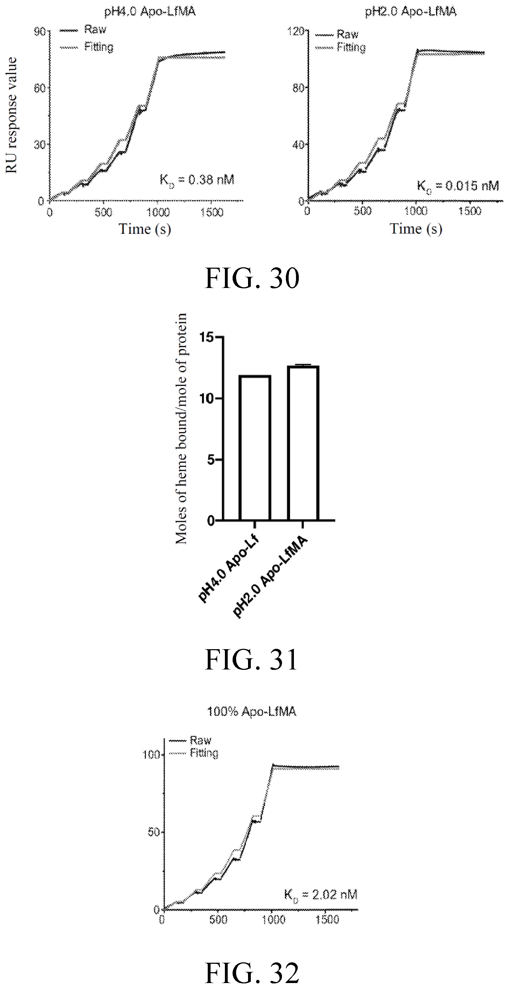

As shown in FIG. 30 and FIG. 31, a binding constant K_{D} of a methacryloylated lactoferrin obtained at pH=2.0 binding to heme and the amount of heme bound to the lactoferrin are both higher than a binding constant K_{D} of a methacryloylated lactoferrin obtained at pH=4.0 binding to heme and the amount of heme bound to the lactoferrin.

### Embodiment 7

A use of a lactoferrin in binding to heme is provided, and specific steps are as follows.
(1) Acetic acid, sodium acetate (at a molar ratio of 1:1), and EDTA are added to a PBS solution with a pH of 2.0 to prepare a mixed solution, and lactoferrin powder to be de-ironed (Bega, Australia) is then dissolved in the mixed solution at room temperature to prepare a lactoferrin solution to be de-ironed with a concentration of 10 g/100 mL.

In the mixed solution, the concentration of PBS is 0.01 M, the total concentration of acetic acid and sodium acetate is 0.1 M, and the concentration of EDTA is 500 mM.

### (2) Preparation of an apo-lactoferrin

The lactoferrin solution to be de-ironed obtained in step (1) is placed into a dialysis bag, the dialysis bag is immersed in a PBS solution for dialysis for 72 h, and retentate obtained after dialysis is first pre-frozen at a temperature of -50°C and then lyophilized under a vacuum of 1 Pa to obtain an apo-lactoferrin with iron content of 0.0139mg/g.

A molecular weight cutoff of the dialysis bag is 7,000 Da. The concentration of the PBS solution for dialysis is gradually reduced. From 0 h to 24 h, the concentration of the PBS solution is gradually reduced from 0.1 M to 0.05 M; from 24 h to 48 h, the concentration of the PBS solution is gradually reduced from 0.05 M to 0.01 M; and from 48 h to 72 h, the concentration of the PBS solution is gradually reduced from 0.01 M to 0 M.
(3) The apo-lactoferrin obtained in step (2) is dissolved in a PBS solution with a pH of 2.0 at room temperature to prepare an apo-lactoferrin stock solution with a concentration of 10 g/100 mL.
(4) Under high-speed stirring at 40 rpm, methacrylic anhydride is added to the apo-lactoferrin stock solution, the pH is then adjusted to 7, reaction is carried out under continuous stirring at 25 °C for 2 h, after the reaction, dialysis is performed for 72 h, after concentration by ultrafiltration, filtering is carried twice through a filtration membrane with a pore size of 0.22 µm, and a filtrate is lyophilized at a temperature of -50°C under a vacuum of 1 Pa to obtain a methacryloylated apo-lactoferrin.

A usage ratio of methacrylic anhydride to the apo-lactoferrin is 2 mL/1 g. A grafting degree of methacrylic anhydride on the methacryloylated apo-lactoferrin is 100%.

The prepared methacryloylated lactoferrin is dissolved in normal saline to prepare a solution with a concentration of 10 mg/mL, which is then injected for binding to free heme.

The methacryloylated lactoferrin has 10 heme-binding sites. A K_{D} value of the lactoferrin binding to heme is 2.02 nM.

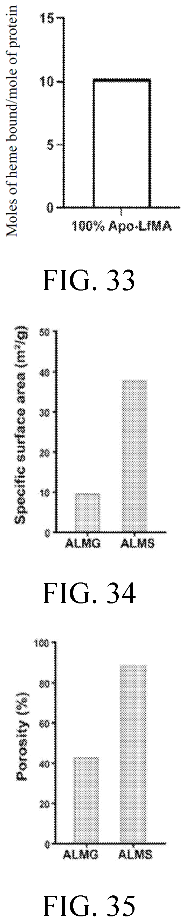

As shown in FIG. 32 and FIG. 33, a binding constant K_{D} of an Apo-LfMA with a methacrylic anhydride modification degree of 100% binding to heme is higher than that of an Apo-LfMA with a grafting degree of 30% binding to heme, indicating that the affinity of the Apo-LfMA with the modification degree of 100% is lower than that of the Apo-LfMA with the grafting degree of 30%, and the quantity of heme-binding sites of the Apo-LfMA with the methacrylic anhydride modification degree of 100% is lower than that of the Apo-LfMA with the grafting degree of 30%.

### Embodiment 8: preparation of an ALMS formulation

An aqueous phase includes a 12% w/v methacryloylated apo-lactoferrin (Apo-LfMA), 3% w/v methacryloylated gelatin (GelMA, for synthesis of GelMA in this application, refer to literature: Zhang, L. et al. Multileveled hierarchical hydrogel with continuous biophysical and biochemical gradients for enhanced repair of full-thickness osteochondral defect. Adv. Mater.35, 2209565 (2023)), and a 0.3% w/v LAP photoinitiator. An oil phase is liquid paraffin including a 5% w/v Span 80 emulsifier.

The aqueous phase and the oil phase are each driven by a syringe pump. The oil phase flows through a channel at a rate of 3 mL/min, the aqueous phase flows through the channel at a rate of 100 µL/min, and the oil phase and the aqueous phase are emulsified to form droplets. The collected droplets are first frozen at -80°C for 1 min, then frozen in liquid nitrogen, and subsequently undergo photo-crosslinking under ultraviolet light at 405 nm for 3 min. An obtained microsphere is washed three times respectively with petroleum ether, acetone at -80°C, and deionized water, then lyophilized, and stored at 4 °C.

### Embodiment 9: preparation of an ALMG formulation

An aqueous phase includes a 12% w/v methacryloylated apo-lactoferrin (Apo-LfMA), 3% w/v methacryloylated gelatin (GelMA), and a 0.3% w/v LAP photoinitiator. An oil phase is liquid paraffin including a 5% w/v Span 80 emulsifier.

The aqueous phase and the oil phase are each driven by a syringe pump. The oil phase flows through a channel at a rate of 3 mL/min, the aqueous phase flows through the channel at a rate of 100 µL/min, and the oil phase and the aqueous phase are emulsified to form droplets. The emulsion droplets are collected in a 10 cm culture dish, placed at 4 °C for 10 min to maintain their morphology, and then undergo photo-crosslinking under ultraviolet light at 405 nm for 3 min. An obtained microsphere is washed three times respectively with petroleum ether, acetone at -80°C, and deionized water, then lyophilized, and stored at 4 °C.

### Comparative example: preparation of GMG

An aqueous phase includes 12% w/v methacryloylated gelatin (GelMA) and a 0.3% w/v LAP photoinitiator. An oil phase is liquid paraffin including a 5% w/v Span 80 emulsifier.

The aqueous phase and the oil phase are each driven by a syringe pump. The oil phase flows through a channel at a rate of 3 mL/min, the aqueous phase flows through the channel at a rate of 100 µL/min, and the oil phase and the aqueous phase are emulsified to form droplets. The emulsion droplets are collected in a 10 cm culture dish, placed at 4 °C for 10 min to maintain their morphology, and then undergo photo-crosslinking under ultraviolet light at 405 nm for 3 min. An obtained microsphere is washed three times respectively with petroleum ether, acetone at -80°C, and deionized water, then lyophilized, and stored at 4 °C.

### Characterization of ALMS and ALMG

After ALMS and ALMG are lyophilized, a mercury intrusion porosimeter (Micromeritics Auto Pore IV 9510, Micromeritics Instrument Corporation, USA) is used to measure the porosity and specific surface areas of ALMG and ALMS. FIG. 34 shows the specific surface areas of ALMG and ALMS, and FIG. 35 shows the porosity of ALMG and ALMS.

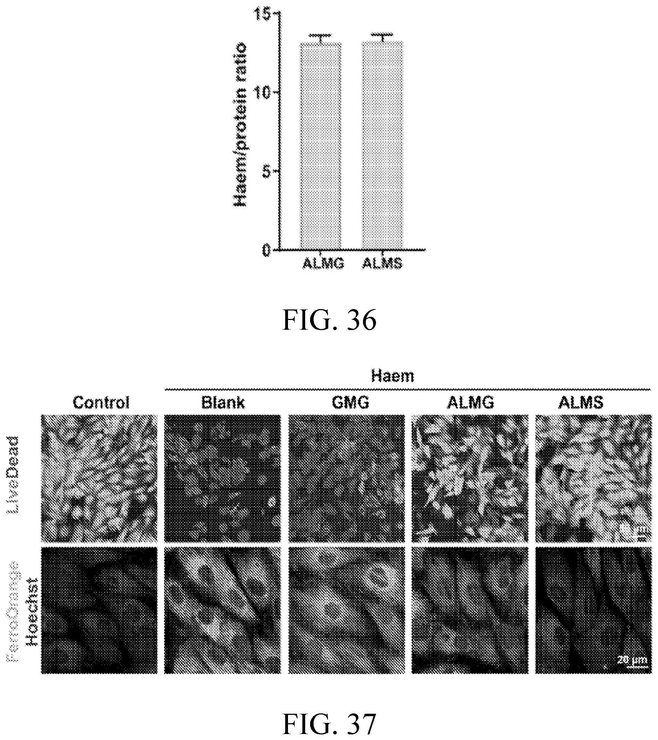

ALMG and ALMS are incubated in a 250 µM/L heme solution at pH=7.2. Aqueous phase samples are collected at 30 min, 1 h, 2 h, 4 h, 8 h, and 24 h. Heme content is evaluated by using an oxalic acid method and an inductively coupled plasma (ICP) spectrometer to determine heme adsorption capacities of ALMG and ALMS. FIG. 36 shows the amounts of ALMG- and ALMS-bound saturated heme (heme/protein ratio) measured by extracting bound heme.

### In vitro experimental data of LMS, ALMG, and GMG

FIG. 37 shows a live/dead staining condition and an intracellular ferrous ion (Fe²⁺, stained with Ferro Orange) condition of cardiomyocytes treated with heme for 24 h in the presence of GMG, ALMG, or ALMS. In the figure, green represents live cells, and red represents dead cells.

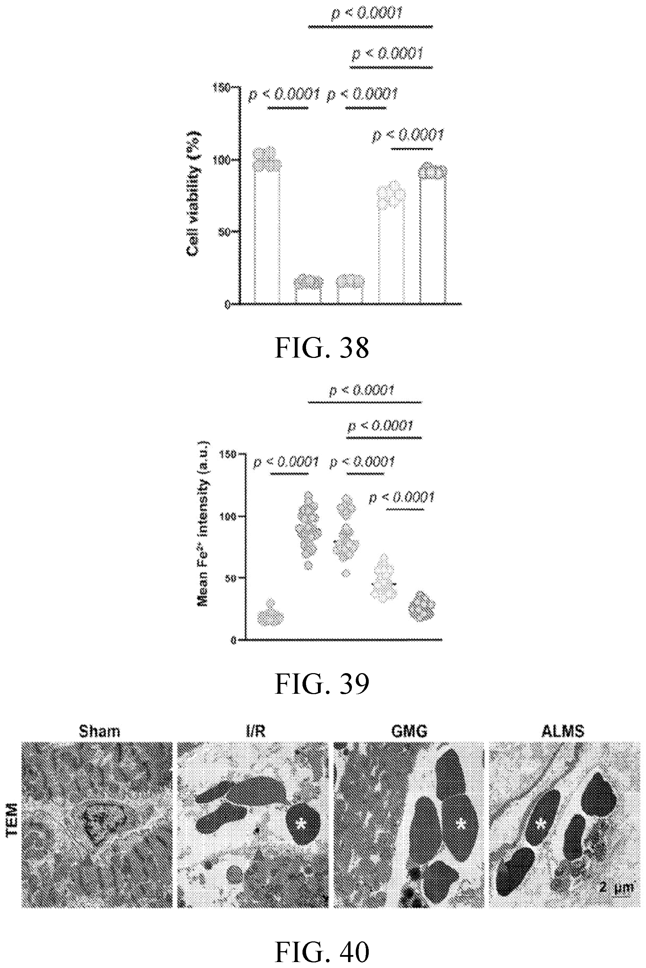

FIG. 38 shows the cell viability of cardiomyocytes (H9C2) treated with heme for 24 h in the presence of GMG, ALMG, or ALMS (n=6). FIG. 39 shows the average fluorescence intensity of intracellular ferrous ions (Fe²⁺) in cardiomyocytes treated with heme for 24 h in the presence of GMG, ALMG, or ALMS (n=6).

In FIG. 38 and FIG. 39, a control group is a culture medium without heme, and a blank group (Haem) is a culture medium including only heme.

### In vivo experimental data of ALMS, ALMG, and GMG

In a rat ischemia/reperfusion (I/R) model, ALMS inhibits myocardial ferroptosis induced by intramyocardial hemorrhage (IMH).

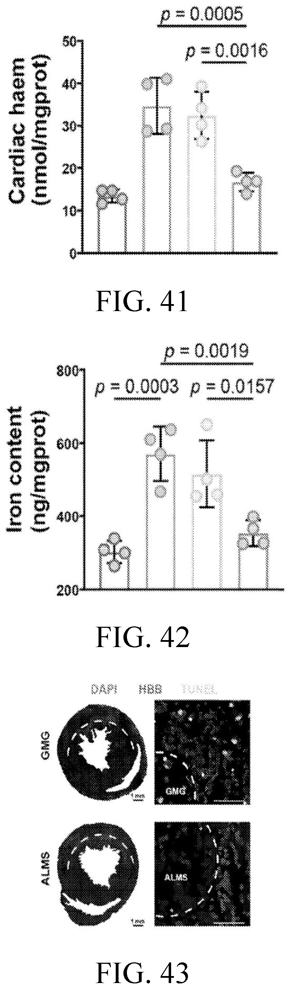

As shown in FIG. 40, the heart of an I/R rat with an IMH phenotype is analyzed by means of pathological and transmission electron microscopic (TEM) observation. As shown in FIG. 41 and FIG. 42, in the rat I/R model, IMH leads to rapid accumulation of free heme in left ventricular myocardium (cardiac heme) to 34.74±5.70 nmol/mg prot within the first 24 h, which increases myocardial iron content by 89%. Local implantation of ALMS reduces free heme in the myocardium to 16.69±1.87 nmol/mg prot, which is not significantly different from a baseline level in a sham-operated group. Heme concentration reduction leads to an 81% decrease in iron content.

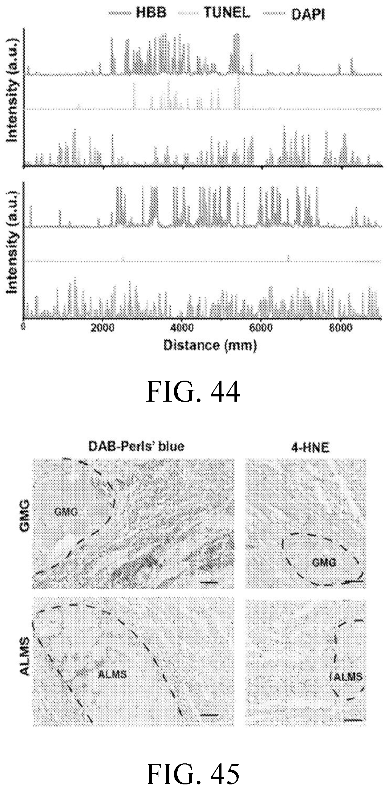

As shown in FIG. 41, in a GMG group, heme is not guided into an implanted microsphere, and therefore, a heme concentration and a cardiomyocyt loss are not reduced. As shown in FIG. 43 and FIG. 44, in the GMG group and an I/R group, hemoglobin β-chain (HBB)-positive red blood cells are spatially associated with terminal -deoxynucleotidyl transferase mediated nick end labeling (TUNEL) signals. However, in an ALMS-treated rat, TUNEL signals in an HBB-positive region are sparse and weak, spatially demonstrating the impact of IMH on I/R injury and the rescuing effect of ALMS.

As shown in FIG. 45, both GMG and ALMS microspheres are aggregated at an injection site, but only ALMS serves as a site for guiding exogenous heme from IMH. Iron signals are concentrated at the ALMS injection site rather than in surrounding tissue, whereas severe iron deposition is clearly observed in surrounding tissue where GMG is implanted.

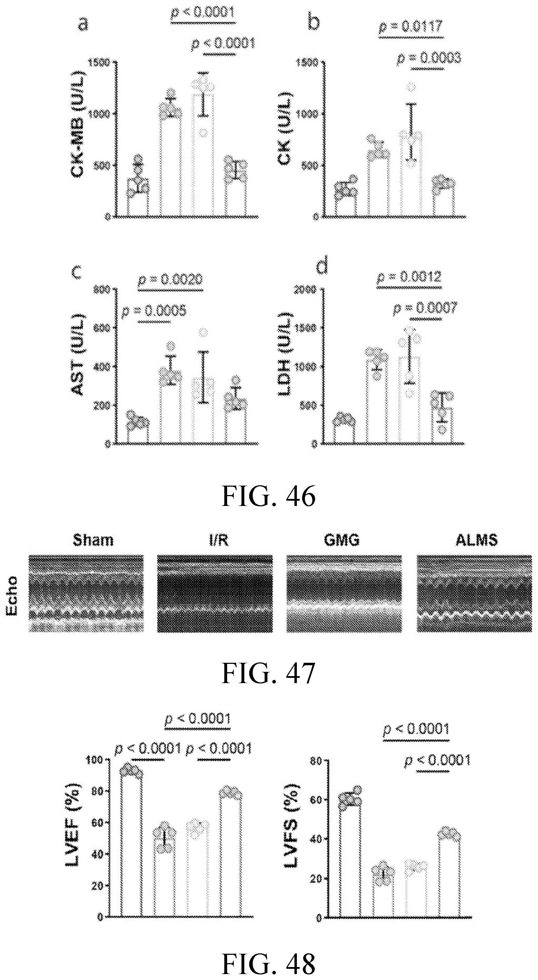

As shown in FIG. 46, serum levels of myocardial injury markers (including creatine kinase isoenzyme MB (CK-MB), creatine kinase (CK), aspartate aminotransferase (AST), and lactate dehydrogenase (LDH)) in the ALMS group are maintained at the same levels as those in the sham-operated group, confirming that ALMS reduces I/R injury by mitigating heme-induced ferroptosis.

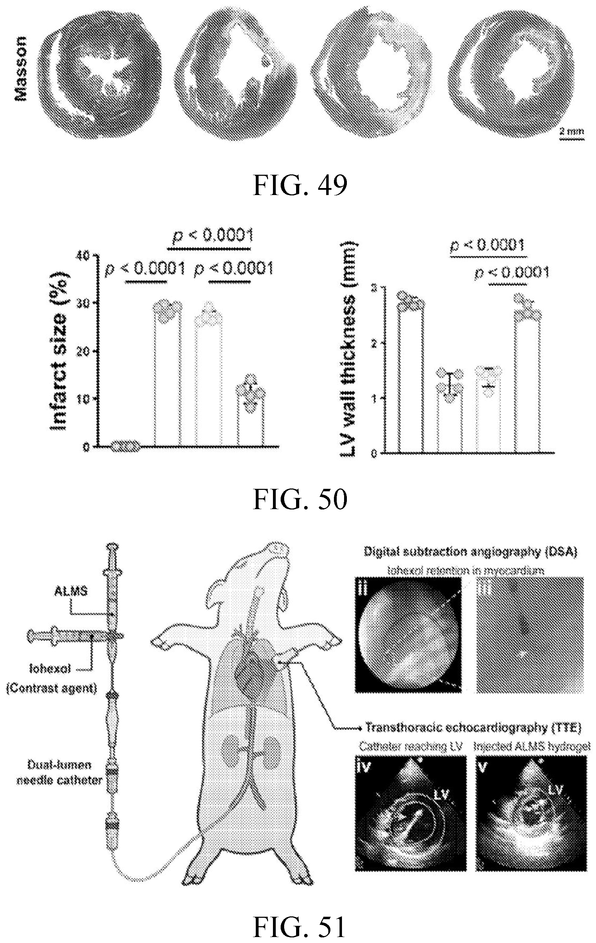

As shown in FIG. 47 and FIG. 48, long-term echocardiographic assessment at 35 days post-surgery demonstrates that, compared with the GMG group and the I/R group, ALMS attenuates left ventricular remodeling in terms of cardiac functions (left ventricular ejection fraction (LVEF) and left ventricular fractional shortening (LVFS)) and cardiac geometry (left ventricular internal dimension in systole (LVIDS) and left ventricular end-systolic volume (LVESV)). As also shown in FIG. 49 and FIG. 50, compared with the I/R group, ALMS reduces a fibrotic area of an infarcted region by 17.3% and maintains a left ventricular wall thickness.

In a porcine cardiac ischemia/reperfusion model, the safety and efficacy of ALMS in treating an iron overload-related disease (for example, myocardial ischemia-reperfusion injury, renal ischemia-reperfusion injury, or cerebral infarction-reperfusion injury) are further demonstrated. ALMS is compatible with a minimally invasive transcatheter injection system (TEISOI, Dinova Medtech), which has been used for transendocardial hydrogel injection in patients with heart failure. As shown in FIG. 51, under guidance of digital subtraction angiography (DSA), a steerable dual-lumen needle catheter is inserted through a femoral artery, and passes through the aortic valve to the left ventricular endocardium. An injection site is determined by transthoracic echocardiography and the digital subtraction angiography (refer to FIG. 51 (ii-iv)). ALMS is injected into the left ventricular wall, without leakage, bleeding, or ventricular wall perforation (refer to FIG. 51(v)). Aggregated ALMS deposition is observed in the myocardium, and all pigs survive procedures.

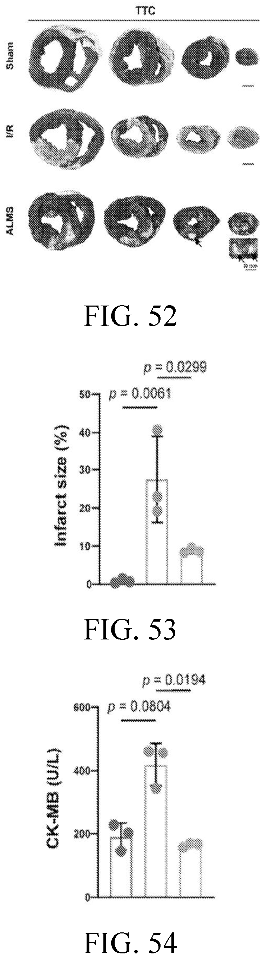

In an efficacy study, ALMS is injected into an infarcted left ventricle at nine evenly distributed sites, followed immediately by reperfusion. Three days after surgery, as shown in FIG. 52, TTC staining reveals extensive necrotic myocardium in the anterior wall of the left ventricle and the interventricular septum. It should be noted that compared with the ischemia/reperfusion control group, the ALMS group exhibits a significantly smaller volume of necrotic myocardium, and myocardium surrounding the ALMS injection site shows a color closer to that of healthy myocardium.

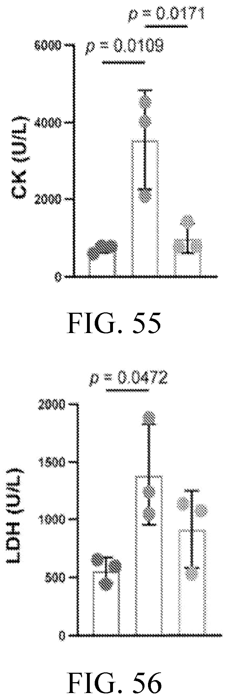

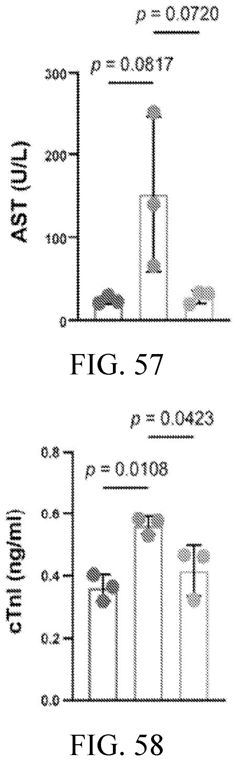

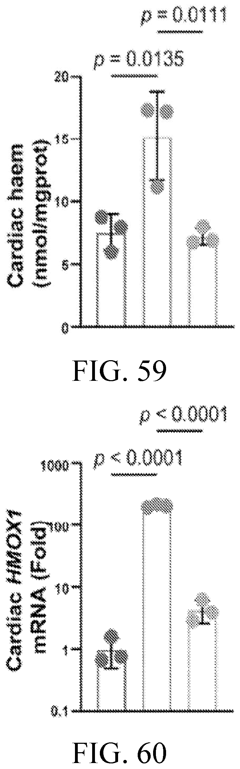

As shown in FIG. 53, in comparison with the ischemia/reperfusion group, the volume of necrotic myocardium in the ALMS group is reduced by 70.4%. As shown in FIG. 54 to FIG. 58, implantation of ALMS also adjusts elevations of serum cardiac enzyme markers (creatine kinase isoenzyme MB (CK-MB), creatine kinase (CK), lactate dehydrogenase (LDH), and aspartate aminotransferase (AST)) and cardiac troponin I (cTnI) following ischemia/reperfusion injury. As shown in FIG. 59, a free heme level in the ischemia/reperfusion group increases to 2.02 times that of healthy myocardium, while ALMS reduces the heme level to a healthy level by capturing exogenous heme. As shown in FIG. 60, in the ischemia/reperfusion (I/R) group, an mRNA level of heme oxygenase 1 (HMOX1) is significantly upregulated, indicating that heme has been internalized into intracellular iron, and ALMS significantly reduces heme oxygenase 1 by capturing exogenous heme.

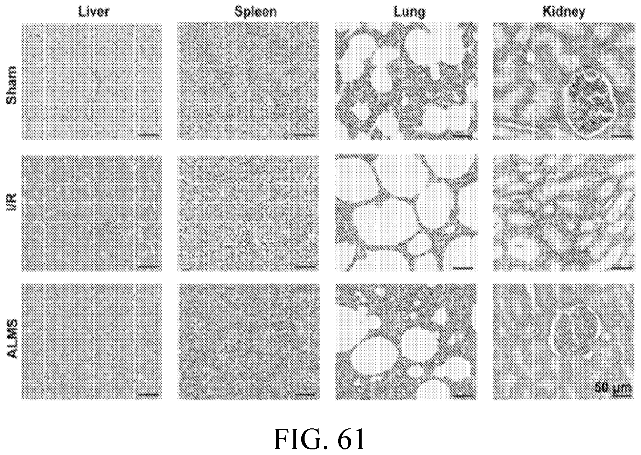

In a clinically relevant large animal model, ALMS inhibits ferroptosis induced by intramyocardial hemorrhage and ischemia/reperfusion injury. As shown in FIG. 61, hematoxylin-eosin analysis of major organs confirms biological safety of ALMS for in vivo treatment.

## Claims

1. A lactoferrin having a strong iron-absorbing capability, wherein iron saturation content of the lactoferrin having the strong iron-absorbing capability is above 10 mg/g.

2. The lactoferrin having the strong iron-absorbing capability according to claim 1, wherein the iron saturation content of the lactoferrin having the strong iron-absorbing capability ranges from 10 mg/g to 14.43 mg/g.

3. The lactoferrin having the strong iron-absorbing capability according to claim 1, wherein the lactoferrin having the strong iron-absorbing capability is obtained by methacryloylating a lactoferrin with low iron content.

4. The lactoferrin having the strong iron-absorbing capability according to claim 3, wherein the lactoferrin with the low iron content refers to a lactoferrin with iron content less than 0.15 mg/g.

5. The lactoferrin having the strong iron-absorbing capability according to claim 3, wherein the iron content of the lactoferrin with the low iron content ranges from 0.0139 mg/g to 0.15 mg/g.

6. A lactoferrin having a strong iron-absorbing capability, wherein the lactoferrin having the strong iron-absorbing capability has 14 to 20 iron ion-binding sites.

7. A use of a lactoferrin in preparation of an iron chelator, wherein the lactoferrin is the lactoferrin having the strong iron-absorbing capability according to any one of claims 1 to 6.

8. A use of a lactoferrin in reducing iron content in a microenvironment, wherein the lactoferrin is the lactoferrin having the strong iron-absorbing capability according to any one of claims 1 to 6.

9. A use of a lactoferrin in preparation of a therapeutic agent for an iron overload disease, wherein the lactoferrin is the lactoferrin having the strong iron-absorbing capability according to any one of claims 1 to 6.

10. The use according to claim 9, wherein the iron overload disease is myocardial ischemia-reperfusion injury, renal ischemia-reperfusion injury, or cerebral infarction-reperfusion injury.

11. A lactoferrin, wherein the lactoferrin is a methacryloylated apo-lactoferrin, and the lactoferrin has 7 to 13 heme-binding sites.

12. The lactoferrin according to claim 11, wherein iron content of the apo-lactoferrin is less than 0.15 mg/g.

13. The lactoferrin according to claim 11, wherein methacryloylation is performed using methacrylic anhydride, wherein a grafting ratio of methacrylic anhydride ranges from 30% to 100%.

14. A lactoferrin, wherein a K_{D} value of the lactoferrin binding to heme ranges from 0.015 nM to 2.02 nM.

15. A use of the lactoferrin according to any one of claims 11 to 14, wherein the lactoferrin is used for binding to heme.

16. The use of the lactoferrin according to claim 15, wherein the lactoferrin is prepared as a solution or hydrogel and then injected to bind to free heme.

17. The use of the lactoferrin according to claim 16, wherein preparing the lactoferrin as the solution comprises:
dissolving the lactoferrin in normal saline to prepare a solution with a concentration ranging from 5 mg/mL to 10 mg/mL.

18. The use of the lactoferrin according to claim 16, wherein preparing the lactoferrin as the hydrogel comprises:
lyophilizing the lactoferrin to obtain lyophilized powder, dissolving the lyophilized powder in a PBS solution to prepare a stock solution, adding an initiator, and performing curing to form the hydrogel; and
the initiator is a free radical initiator, a redox initiator, a vitamin C-hydrogen peroxide system, or a glutathione-peroxide system.

19. The use of the lactoferrin according to claim 18, wherein the free radical initiator is selected from at least one of Irgacure 2959, Irgacure 819, and camphorquinone.

20. The use of the lactoferrin according to claim 18, wherein the redox initiator comprises:
an oxidizing agent, selected from at least one of ammonium persulfate, potassium persulfate, and sodium persulfate; and
a reducing agent, selected from at least one of ascorbic acid, TEMED, sodium sulfite, and sodium bisulfite.

21. The use of the lactoferrin according to claim 18, wherein the vitamin C-hydrogen peroxide system comprises:
an oxidizing agent: H₂O₂; and
a reducing agent: ascorbic acid.

22. The use of the lactoferrin according to claim 18, wherein the glutathione-peroxide system comprises:
an oxidizing agent: urea peroxide; and
a reducing agent: glutathione.

23. The use of the lactoferrin according to claim 18, wherein the initiator is a photoinitiator, namely, lithium phenyl-2,4,6-trimethylbenzoylphosphinate, and curing is performed under light to form a hydrogel.

24. The use of the lactoferrin according to claim 23, wherein in the stock solution, the concentration of the lactoferrin is 0.12 g/mL, and the amount of lithium phenyl-2,4,6-trimethylbenzoylphosphinate is 0.4 wt%.

25. The use of the lactoferrin according to claim 16, wherein an injection mode is selected from at least one of intravenous injection, intraperitoneal injection, and in-situ tissue injection.

26. A preparation method for a lactoferrin having a strong iron-absorbing capability, comprising: methacryloylating a lactoferrin with low iron content to obtain the lactoferrin having the strong iron-absorbing capability.

27. The preparation method according to claim 26, wherein the lactoferrin with the low iron content is methacryloylated using methacrylic anhydride.

28. The preparation method according to claim 27, wherein a usage ratio of methacrylic anhydride to the lactoferrin with the low iron content ranges from 0.01 mL/1 g to 2 mL/1 g.

29. The preparation method according to claim 28, wherein the usage ratio of methacrylic anhydride to the lactoferrin with the low iron content is 0.03 mL/1 g.

30. The preparation method according to claim 27, wherein during methacryloylation, a grafting ratio of methacrylic anhydride ranges from 10% to 100%.

31. The preparation method according to claim 30, wherein during methacryloylation, the grafting ratio of methacrylic anhydride ranges from 30% to 100%.

32. The preparation method according to claim 26, wherein the preparation method further comprises de-ironing a lactoferrin to obtain the lactoferrin with the low iron content.

33. The preparation method according to claim 32, wherein the lactoferrin with the low iron content refers to a lactoferrin with iron content less than 0.15 mg/g; and iron saturation content of the lactoferrin having the strong iron-absorbing capability is above 10 mg/g.

34. The preparation method according to claim 33, wherein the iron content of the lactoferrin with the low iron content ranges from 0.010 mg/g to 0.015 mg/g.

35. The preparation method according to claim 33, wherein the iron content of the lactoferrin with the low iron content is 0.0139 mg/g.

36. The preparation method according to claim 32, wherein the ratio of the iron content of the lactoferrin with the low iron content to that of the lactoferrin before de-ironing ranges from 3.97% to 9.8%; and the ratio of iron content of the lactoferrin having the strong iron-absorbing capability to that of the lactoferrin before de-ironing ranges from 2.86% to 8.5%.

37. The preparation method according to claim 32, wherein the ratio of the iron content of the lactoferrin with the low iron content to that of the lactoferrin before de-ironing is 9.8%; and the ratio of iron content of the lactoferrin having the strong iron-absorbing capability to that of the lactoferrin before de-ironing is 8.5%.

38. The preparation method according to claim 32, wherein during lactoferrin de-ironing, a lactoferrin solution is first prepared, and after dialysis, retentate is lyophilized to obtain the lactoferrin with the low iron content.

39. The preparation method according to claim 38, wherein the lactoferrin solution is prepared by dissolving lactoferrin powder in a mixed solution at room temperature.

40. The preparation method according to claim 39, wherein the mixed solution is prepared by adding acetic acid, sodium acetate, and EDTA to a PBS solution with a pH ranging from 2.0 to 4.0.

41. The preparation method according to claim 40, wherein a molar ratio of acetic acid to sodium acetate is 1:1.

42. The preparation method according to claim 39, wherein in the mixed solution, the concentration of PBS is 0.01 M, the total concentration of acetic acid and sodium acetate is 0.1 M, and the concentration of EDTA ranges from 40 mM to 500 mM.

43. The preparation method according to claim 38, wherein the concentration of the lactoferrin solution ranges from 0.5 g/100 mL to 10 g/100 mL.

44. The preparation method according to claim 38, wherein during lactoferrin solution dialysis, the method comprises placing the lactoferrin solution into a dialysis bag, and placing the dialysis bag in a PBS solution for dialysis for 48 h to 72 h.

45. The preparation method according to claim 44, wherein during dialysis, the concentration of the PBS solution in which the dialysis bag is placed is gradually reduced.

46. The preparation method according to claim 45, wherein the concentration of the PBS solution is gradually reduced in the following manner:
from 0 h to 24 h (excluding 24 h), the concentration of the PBS solution is gradually reduced from 0.1 M to 0.05 M;
from 24 h to 48 h (excluding 48 h), the concentration of the PBS solution is gradually reduced from 0.05 M to 0.01 M; and
from 48 h to 72 h, the concentration of the PBS solution is gradually reduced from 0.01 M to 0 M.

47. The preparation method according to claim 26, wherein the process of methacryloylating a lactoferrin with low iron content comprises:
step (1): dissolving the lactoferrin with the low iron content in a PBS solution at room temperature to prepare a lactoferrin stock solution; and
step (2): adding methacrylic anhydride to the lactoferrin stock solution for reaction to obtain the lactoferrin having the strong iron-absorbing capability.

48. The preparation method according to claim 47, wherein in step (1), the lactoferrin with the low iron content is dissolved in the PBS solution at room temperature to prepare a lactoferrin stock solution with a concentration ranging from 1 g/100 mL to 10 g/100 mL.

49. The preparation method according to claim 48, wherein in step (1), the PBS solution has a pH value ranging from 2.0 to 4.0.

50. The preparation method according to claim 47, wherein in step (2), a usage ratio of methacrylic anhydride to the lactoferrin with the low iron content ranges from 0.01 mL/1 g to 2 mL/1 g.

51. The preparation method according to claim 50, wherein in step (2), the usage ratio of methacrylic anhydride to the lactoferrin with the low iron content is 0.03 mL/1 g.

52. The preparation method according to claim 47, wherein in step (2), during methacryloylation, a grafting ratio of methacrylic anhydride ranges from 10% to 100%.

53. The preparation method according to claim 47, wherein in step (2), methacrylic anhydride is added to the lactoferrin stock solution under high-speed stirring at 400 to 1,000 rpm.

54. The preparation method according to claim 47, wherein in step (2), after methacrylic anhydride is added to the lactoferrin stock solution, a pH value is adjusted to 6.5 to 7.5 for the reaction.

55. The preparation method according to claim 54, wherein in step (2), after the pH value is adjusted to 6.5 to 7.5, the reaction is carried out under continuous stirring at 10 to 25 °C for 0.5 h to 24 h, and after the reaction, post-treatment is performed to obtain the lactoferrin having the strong iron-absorbing capability.

56. The preparation method according to claim 55, wherein the post-treatment involves dialysis for 24 h to 72 h, concentration by ultrafiltration, then filtration twice through a filtration membrane, and lyophilization of a filtrate to obtain the lactoferrin having the strong iron-absorbing capability.

57. The preparation method according to claim 56, wherein the filtration membrane used for the filtration has a pore size of 0.1 µm or 0.22 µm.

58. A formulation for removing heme, wherein the formulation comprises the lactoferrin according to any one of claims 1 to 5 or claims 11 to 14.

59. The formulation for removing heme according to claim 58, wherein the formulation is a microsphere with a porous structure, and the porosity of the formulation is greater than 80%.

60. The formulation for removing heme according to claim 58, wherein the specific surface area of the formulation is greater than 35 m²/g.

61. The formulation for removing heme according to claim 58, wherein the formulation has 7 to 13 lactoferrin-binding sites.

62. A preparation method for a formulation for removing heme, wherein the formulation is the formulation for removing heme according to any one of claims 58 to 61, the preparation method comprises mixing an aqueous phase and an oil phase to form an emulsion comprising a photoinitiator, and performing photo-crosslinking on the emulsion to obtain the formulation in the form of a microsphere, in the aqueous phase, water is used as a solvent, and the aqueous phase comprises the photoinitiator, the lactoferrin, and methacryloylated gelatin, and in the oil phase, liquid paraffin is used as a solvent, and the oil phase comprises an emulsifier.

63. The preparation method according to claim 62, wherein in the aqueous phase, the content of the lactoferrin ranges from 5% w/v to 30% w/v.

64. The preparation method according to claim 62, wherein in the aqueous phase, the content of methacryloylated gelatin ranges from 1% w/v to 20% w/v.

65. The preparation method according to claim 62, wherein in the aqueous phase, the content of the photoinitiator ranges from 0.1% w/v to 1% w/v.

66. The preparation method according to claim 62, wherein in the aqueous phase, the photoinitiator is lithium phenyl-2,4,6-trimethylbenzoylphosphinate.

67. The preparation method according to claim 62, wherein in the oil phase, the content of the emulsifier ranges from 1% w/v to 20% w/v.

68. The preparation method according to claim 62, wherein in the oil phase, the emulsifier is Span 80.

69. The preparation method according to claim 62, wherein the aqueous phase and the oil phase are each driven by a syringe pump, and the oil phase and the aqueous phase are merged through a channel at a flow rate of 10,000 to 50,000:1 and are emulsified to form droplets.

70. The preparation method according to claim 69, wherein a flow rate of the oil phase ranges from 1 mL/min to 5 mL/min.

71. The preparation method according to claim 69, wherein a flow rate of the aqueous phase ranges from 10 µL/min to 1,000 µL/min.

72. The preparation method according to claim 62, wherein photo-crosslinking is performed on the emulsion under ultraviolet light at 405 nm after the emulsion is frozen.

73. The preparation method according to claim 62, wherein before photo-crosslinking, the emulsion is frozen, wherein the emulsion is frozen at -100°C to -30°C, and then frozen in liquid nitrogen.

74. The preparation method according to claim 62, wherein before photo-crosslinking, the emulsion is placed at 4 °C for 5 min to 10 min to maintain its morphology.

75. The preparation method according to claim 62, wherein after photo-crosslinking, the obtained microsphere is washed with petroleum ether, acetone at -80 °C, and deionized water to obtain the formulation.

76. A use of a formulation in a therapeutic agent for an iron overload disease, wherein the formulation is the formulation for removing heme according to any one of claims 58 to 61.

77. The use according to claim 76, wherein the iron overload disease is myocardial ischemia-reperfusion injury, renal ischemia-reperfusion injury, or cerebral infarction-reperfusion injury.

78. A therapy method for an iron overload-related disease, comprising: injecting the formulation according to any one of claims 58 to 61 into a lesion site.

79. The therapy method according to claim 78, wherein the iron overload-related disease includes myocardial ischemia-reperfusion injury, renal ischemia-reperfusion injury, or cerebral infarction-reperfusion injury.

80. The therapy method according to claim 78, wherein the formulation is injected into the lesion site using an interventional device comprising a needle catheter.

81. A reperfusion therapy method, comprising:
injecting the formulation according to any one of claims 58 to 61 into ischemic tissue; and
performing reperfusion therapy on the ischemic tissue.

82. The reperfusion therapy method according to claim 81, comprising: pre-injecting the formulation into the ischemic tissue, and then performing reperfusion therapy.

83. A reperfusion therapy method for myocardial ischemia, comprising: pre-injecting the formulation according to any one of claims 58 to 61 into a left ventricular wall, and then performing reperfusion therapy on the left ventricle.

84. The reperfusion therapy method according to claim 83, wherein the formulation is injected into the left ventricular wall using an interventional device comprising a needle catheter, wherein the needle catheter sequentially passes through a femoral artery and an aortic valve to reach a left ventricular endocardium, and the formulation is injected into the left ventricular wall through the needle catheter.

85. The reperfusion therapy method according to claim 84, wherein injection points of the needle catheter are distributed at predetermined sites on the left ventricular wall, and there are at least three injection points.

86. The reperfusion therapy method according to claim 85, wherein an injection volume at each injection point is not less than 0.1 mL.
